(19) Europäisches Patentamt
European Patent Office
Office européen des brevets

(11) **EP 4 270 160 B1**

(12) **EUROPEAN PATENT SPECIFICATION**

(45) Date of publication and mention
of the grant of the patent:
**04.06.2025 Bulletin 2025/23**

(21) Application number: **23719257.0**

(22) Date of filing: **02.03.2023**

(51) International Patent Classification (IPC):
*G06F 3/01* (2006.01)    *G06F 3/0484* (2022.01)
*H04M 1/72454* (2021.01)    *G06F 3/00* (2006.01)
*A61B 5/00* (2006.01)    *G06F 9/451* (2018.01)
*G06F 1/16* (2006.01)    *G09G 5/10* (2006.01)

(52) Cooperative Patent Classification (CPC):
**G06F 1/1694; A61B 5/165; G06F 3/012;**
**G06F 3/013; G06F 3/017; G06F 9/451;**
**H04M 1/72454;** G06F 2200/1637; G06F 2203/011;
G09G 2320/0626; G09G 2320/0666;
G09G 2340/0435; G09G 2340/0464; G09G 2354/00

(86) International application number:
**PCT/KR2023/002868**

(87) International publication number:
**WO 2023/167506 (07.09.2023 Gazette 2023/36)**

(54) **METHOD FOR REDUCING FATIGUE LEVEL OF EYES AND ELECTRONIC DEVICE THEREFOR**

VERFAHREN ZUR REDUZIERUNG DES AUGENERMÜDUNGSGRADES UND ELEKTRONISCHE
VORRICHTUNG DAFÜR

PROCÉDÉ DE RÉDUCTION DU NIVEAU DE FATIGUE OCULAIRE ET DISPOSITIF
ÉLECTRONIQUE ASSOCIÉ

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**

(30) Priority: **03.03.2022 KR 20220027414**
         **28.04.2022 KR 20220053040**

(43) Date of publication of application:
**01.11.2023 Bulletin 2023/44**

(73) Proprietor: **Samsung Electronics Co., Ltd.**
**Suwon-Si, Gyeonggi-do 16677 (KR)**

(72) Inventors:
• **KIM, Hyeonseong**
**Gyeonggi-do 16677 (KR)**
• **PARK, Jeongmin**
**Gyeonggi-do 16677 (KR)**

(74) Representative: **Arnold & Siedsma**
**Bezuidenhoutseweg 57**
**2594 AC The Hague (NL)**

(56) References cited:
KR-A- 20150 071 344    KR-A- 20160 044 254
KR-B1- 101 540 161    KR-B1- 102 072 788
KR-B1- 102 362 042    US-A1- 2012 092 172
US-B2- 10 775 883

## Description

### [Technical Field]

Various embodiments disclosed in the present document relate to a method for reducing an eye fatigue level and an electronic device thereof.

### [Background Art]

With the recent development of digital technologies, various types of electronic devices such as mobile communication terminals, smart phones, tablet personal computers (PCs), personal digital assistants (PDAs), notebooks, or wearable devices are being utilized. These electronic devices are reaching a stage of mobile convergence that encompasses the functions of other devices. In particular, the electronic device such as the smart phone can provide a call function, a function of sending and receiving messages such as a short message service (SMS), a multimedia message service (MMS), and an electronic mail, a photographing function, a broadcasting function, a video play function, a music play function, an Internet function, a messenger function, a game function, etc.

As the electronic device supports various functions, the amount of time a user faces the electronic device is gradually increasing. For example, to use numerous functions, the users can use the electronic device even while riding a vehicle or walking regardless of time and place.

As the amount of time the user faces the electronic device is increased, and in particular, the amount of time spent using the electronic device during movement is increased, and in order to reduce a user's eye fatigue caused by this, a technology for controlling a frame per seconds (FPS) of a display of the electronic device or delaying a screen output for a predetermined time is being applied.

US10775883 B2 discloses an information processing method, an information processing apparatus and a user equipment. The method comprises: acquiring eye movement information related to that at least one eye of a user gazes a user equipment; and determining relative movement information of the user equipment relative to the head of the user at least according to a non-saccade eye movement part in the eye movement information. Relative movement between a user equipment and the head of a user can be determined according to a non-saccade eye movement part(s) of eye movement information related to the user gazes the user equipment, providing a basis for increasing the accuracy of compensation corresponding to the relative movement.

KR101540161 B1 discloses an apparatus for controlling the position of a display of a wearable device including a display module on which the display is movably mounted; and a motion compensation unit for controlling the position of the display according to the user's movement, so that a smooth image compensation effect can be obtained, the user can accurately check the content displayed on the display, and the user's gaze on the display is focused. It can be fixed in one place and can expand the usage environment of wearable devices by reducing the fatigue felt by users.

### [Disclosure of Invention]

### [Technical Problem]

As the user's use of an electronic device is prolonged, user's eye fatigue can increase, and due to this, a user may suffer adverse effects such as reduced eyesight, dry eyes, congestion, and the like. Also, the eye fatigue is not limited to just the eyes, but can be related to brain activity and cause the decrease of concentration, the decrease of memory, and mental abnormalities.

For example, when the user uses the electronic device while riding in a vehicle or while walking, the user's body is shaken by an external force and due to this, a user's focus is shaken, which can cause dizziness in a short term, and can accumulate a fatigue level in the ciliary muscle in a mid to long term, thereby deteriorating the function of the eye and shorten the time of occurrence of presbyopia.

Various embodiments disclosed in the present document can provide a method and an electronic device for acquiring feature values related to the electronic device and an external electronic device, through communication with the external electronic device, and controlling a display, based on the acquired feature values.

### [Solution to Problem]

The invention is set out in the independent claims 1 and 6. Preferred embodiments are set out in the dependent claims.

According to an aspect a control method of an electronic device according to an exemplary embodiment includes the operations of connecting with an external electronic device worn by a user by using wireless communication, determining whether the user is gazing at the electronic device by using at least one first sensor, acquiring a shaking pattern of the electronic device by using at least one second sensor in response to the user being gazing at the electronic device, acquiring a first feature value and a second feature value which are related to a change of position between the electronic device and the external electronic device, by using at least one third sensor when the acquired shaking pattern corresponds to a predetermined condition, acquiring an eye fatigue level of the user, based on the first feature value and the second feature value, and controlling a display of the electronic device, based on the acquired fatigue level.

According to another aspect an electronic device of according to an exemplary embodiment includes a display, a wireless communication circuit, and at least one processor electrically connected to the wireless communication circuit. The at least one processor may operatively connect with an external electronic device worn by a user by using the wireless communication circuit, determine whether the user is gazing at the display of the electronic device by using at least one first sensor, acquire a shaking pattern of the electronic device by using at least one second sensor in response to the user being gazing at the electronic device, acquire a first feature value and a second feature value which are related to a change of position between the electronic device and the external electronic device by using at least one third sensor when the acquired shaking pattern corresponds to a predetermined condition, acquire an eye fatigue level of the user, based on the first feature value and the second feature value, and control the display, based on the acquired fatigue level.

**[Advantageous Effects of Invention]**

Various embodiments disclosed in the present document may identify a user's eye fatigue level, based on a difference of each axis movement distance change amount and a distance between an electronic device and an external electronic device, and change a display position of a User interface, UI, displayed on a display, based on the identified user's eye fatigue level.

Also, various embodiments may provide a UI for recommending a user to stop using an electronic device, based on a user's eye fatigue level.

Also, various embodiments may provide a UI capable of identifying a cumulative fatigue level, based on a difference of each axis movement distance change amount and a distance between an electronic device and an external electronic device, and receiving a user input at a specified time, based on the identified cumulative fatigue level.

Also, various embodiments may control at least some of a brightness of a display, a scanning rate, and a color, based on a user's eye fatigue level.

In addition to this, various effects identified directly or indirectly through the present document may be provided.

**[Brief Description of Drawings]**

FIG. 1 illustrates an electronic device and an external electronic device connected to the electronic device through wireless communication according to an exemplary embodiment.
FIG. 2 is a flowchart illustrating an operation of acquiring a user's eye fatigue level, and controlling a display, based on the acquired fatigue level, by an electronic device according to an exemplary embodiment.

FIG. 3 is a flowchart illustrating an operation of acquiring a first feature value and a second feature value by an electronic device according to an exemplary embodiment.
FIG. 4A illustrates a movement distance change amount for each axis of an electronic device according to an exemplary embodiment.
FIG. 4B illustrates a movement distance change amount of a direction perpendicular to the ground of an electronic device according to an exemplary embodiment.
FIG. 4C illustrates a movement distance change amount for each axis of an external electronic device according to an exemplary embodiment.
FIG. 4D illustrates a movement distance change amount of a direction perpendicular to the ground of an external electronic device according to an exemplary embodiment.
FIG. 5 is a flowchart illustrating an operation of controlling a display position of a first UI displayed on a display by an electronic device according to an exemplary embodiment.
FIG. 6 illustrates an output control model for a UI of an electronic device according to an exemplary embodiment.
FIG. 7 illustrates a construction in which an electronic device controls a display position of a first UI displayed on a display according to an exemplary embodiment.
FIG. 8 is a flowchart illustrating an operation of acquiring a cumulative fatigue level for a predetermined time and controlling at least some of a brightness of a display, a scanning rate, and a color, based on the acquired cumulative fatigue level, by an electronic device according to an exemplary embodiment.
FIG. 9 is a flowchart illustrating an operation of deactivating an output control model and a UWB antenna when a shaking pattern of an electronic device does not correspond to a predetermined condition according to an exemplary embodiment.
FIG. 10 illustrates a construction of displaying a second UI on a display as a display position of a first UI is changed according to an exemplary embodiment.
FIG. 11 illustrates a construction of displaying a third UI capable of receiving a user's input through a display, based on a cumulative fatigue level, according to an exemplary embodiment.
FIG. 12 illustrates a construction of displaying a fourth UI through a display at a specified time, based on a cumulative fatigue level, according to an exemplary embodiment.
FIG. 13 is a diagram illustrating an electronic device in a network environment according to an exemplary embodiment.

In connection with a description of the drawings, the same or similar reference numerals may be used for the same or similar elements.

**[Best Mode for Carrying out the Invention]**

Hereinafter, various embodiments disclosed in the present document will be described with reference to the accompanying drawings. However, this is not intended to limit the various embodiments of the present disclosure to a specific embodiment, but extends also to other embodiments falling within the scope of the appended claims.

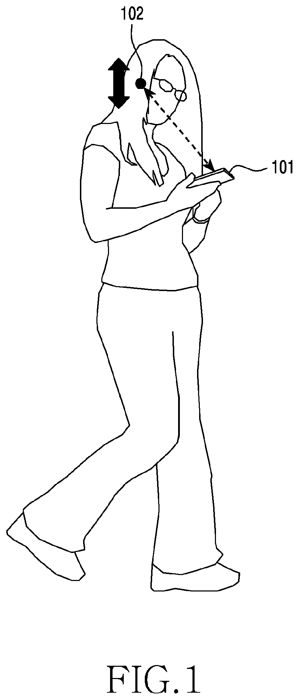

FIG. 1 illustrates an electronic device and an external electronic device connected to the electronic device through wireless communication according to an exemplary embodiment.

Referring to FIG. 1, according to an exemplary embodiment, a user may use an electronic device 101 and an external electronic device 102 connected to the electronic device 101 through wireless communication together. According to an exemplary embodiment, the user may use the electronic device 101 and the external electronic device 102 together while walking. According to another exemplary embodiment (not shown), the user may use the electronic device 101 and the external electronic device 102 together while riding a vehicle.

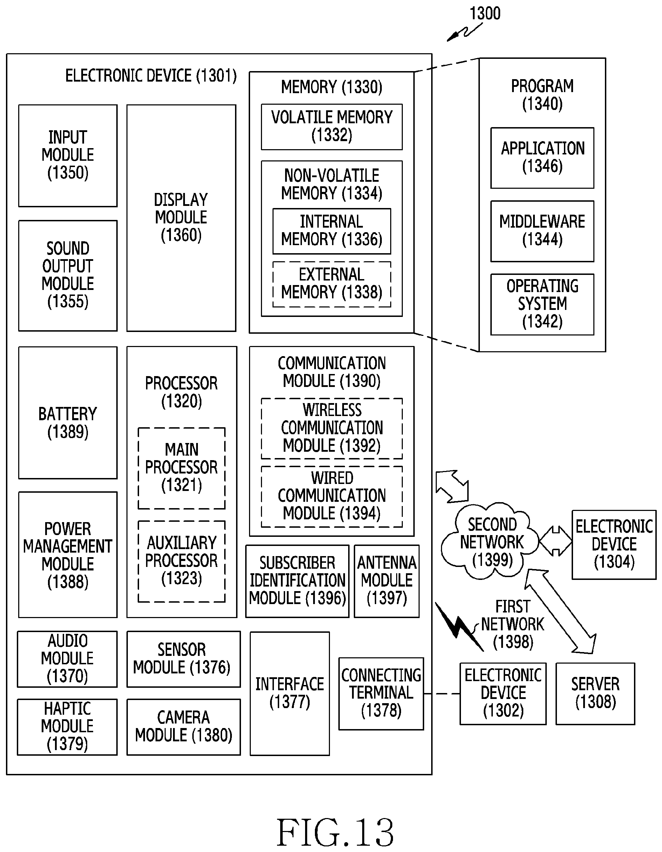

According to an exemplary embodiment, the electronic device 101 may be referred to as various types of devices including a display (e.g., a display 1360 of FIG. 13). The electronic device 101 may be referred to as, for example, a portable communication device (e.g., a smart phone), a portable multimedia device (e.g., a tablet PC), or a portable medical device, but is not limited thereto.

According to another exemplary embodiment, the electronic device 101 may be referred to as a wearable device worn on a user's body. For example, the electronic device 101 may be referred to as a smart watch worn on a user's wrist or a smart ring worn on a user's finger.

According to an exemplary embodiment, the external electronic device 102 may be referred to as a wearable device worn on a user's body. For example, the external electronic device 102 may be referred to as earbuds worn on user's ears. For another example, the external electronic device 102 may be referred to as a head mounted display (HMD) device worn on a user's head. However, the above-described description of the external electronic device 102 is merely exemplary, and the external electronic device 102 may be referred to as various types of wearable devices.

According to an exemplary embodiment, the electronic device 101 may be connected to the external electronic device 102 worn on the user's body through wireless communication. The electronic device 101 may be operatively connected to the external electronic device 102 worn on the user's body through the wireless communication.

According to an exemplary embodiment, the electronic device 101 may be connected to the external electronic device 102 through short-range communication. For example, the electronic device 101 may be connected to the external electronic device 102 through Bluetooth communication. For another example, the electronic device 101 may be connected to the external electronic device 102 through ultra-wide band (UWB) communication. However, a communication method in which the electronic device 101 is connected to the external electronic device 102 is not limited to the above example.

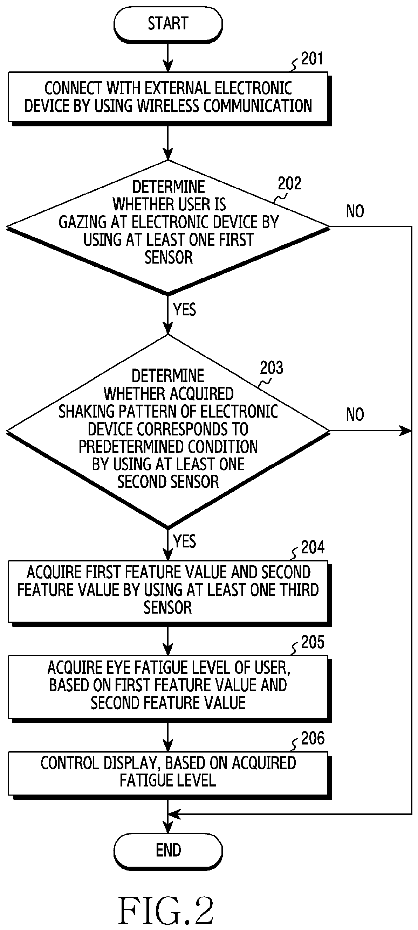

FIG. 2 is a flowchart illustrating an operation of acquiring a user's eye fatigue level, and controlling a display, based on the acquired fatigue level, by an electronic device according to an exemplary embodiment.

Referring to FIG. 2, the electronic device 101 of an exemplary embodiment may acquire a first feature value and a second feature value which are related to a positional change between the electronic device 101 and an external electronic device 102, and control a display, based on a user's eye fatigue level acquired based on the acquired first feature value and second feature value.

According to an exemplary embodiment, in operation 201, the electronic device 101 may connect with the external electronic device 102 by using wireless communication (e.g., Bluetooth communication or UWB communication). According to an exemplary embodiment, in operation 201, the electronic device 101 may connect with the external electronic device 102 worn by a user by using the wireless communication. According to an exemplary embodiment, in operation 201, the electronic device 101 may connect to the external electronic device 102 worn on at least part (e.g., the ear) of the user's body by using the wireless communication.

According to an exemplary embodiment, in operation 202, the electronic device 101 may determine whether a user gazes at the electronic device 101 by using at least one first sensor. According to an exemplary embodiment, in operation 202, the electronic device 101 may determine whether the user gazes at a display of the electronic device 101 by using the at least one first sensor.

According to an exemplary embodiment, in operation 202, the electronic device 101 may determine whether the display is in an activated state.

According to an exemplary embodiment, the at least one first sensor may include a grip sensor and a motion sensor. According to an exemplary embodiment, in operation 202, the electronic device 101 may determine whether the user is holding the electronic device 101 by using the grip sensor. According to an exemplary embodiment, in operation 202, the electronic device 101 may determine whether the display of the electronic device 101 faces a user's face by using the motion sensor. For example, in operation 202, the electronic device 101 may determine whether the display of the electronic device 101 faces the user's face, by detecting a posture of the electronic device 101 by using the motion sensor.

According to an exemplary embodiment, in operation 202, in response to the user being holding the electronic device 101, and the display of the electronic device 101 facing the user's face, the electronic device 101 may detect a distance between the electronic device 101 and the external electronic device 102 by using a UWB antenna.

According to an exemplary embodiment, in operation 202, in response to the user being holding the electronic device 101, and the display of the electronic device 101 facing the user's face, the electronic device 101 may detect a user's eye area by using a camera. For example, the electronic device 101 may detect an area around left and/or right eye including pupils in the user's face as the eye area using the camera.

According to an exemplary embodiment, when the distance between the electronic device 101 and the external electronic device 102 is within a specified distance, and the user's eye area is detected, in operation 202, the electronic device 101 may determine that the user gazes at the electronic device 101.

According to an exemplary embodiment, in response to the user being gazing at the electronic device 101, in operation 203, the electronic device 101 may acquire a shaking pattern of the electronic device 101 by using at least one second sensor. According to an exemplary embodiment, the at least one second sensor may include an acceleration sensor or a gyro sensor. For example, in operation 203, the electronic device 101 may acquire the shaking pattern by using the acceleration sensor or the gyro sensor.

According to an exemplary embodiment, the shaking pattern may refer to a movement distance change amount for at least one axis facing a preset direction within a space where the electronic device 101 exists. A detailed description of this will be made later.

According to an exemplary embodiment, in operation 203, the electronic device 101 may determine whether the shaking pattern acquired using the at least one second sensor corresponds to a predetermined condition. For example, in operation 203, the electronic device 101 may determine whether the shaking pattern acquired using the at least one second sensor corresponds to a shaking pattern of a situation where the user gets in a vehicle. For another example, in operation 203, the electronic device 101 may determine whether the shaking pattern acquired using the at least one second sensor corresponds to a shaking pattern of a situation where the user is walking.

According to an exemplary embodiment, the shaking pattern may be referred to as a frequency component acquired using fast Fourier transform (FFT). According to another exemplary embodiment, the shaking pattern may be referred to as a pattern extracted from a single vector magnitude (SVM) signal.

According to an exemplary embodiment, in operation 204, the electronic device 101 may acquire a first feature value and a second feature value by using at least one third sensor. According to an exemplary embodiment, in response to the shaking pattern of the electronic device 101 acquired through operation 203 corresponding to the predetermined condition, in operation 204, the electronic device 101 may acquire the first feature value and the second feature value by using the at least one third sensor.

According to an exemplary embodiment, the at least one third sensor may include a first inertial sensor. According to an exemplary embodiment, in operation 204, the electronic device 101 may acquire a movement distance change amount for at least one axis (e.g., z-axis) indicating a preset direction (e.g., a direction perpendicular to the ground) in a space where the electronic device 101 is located, by using the first inertial sensor.

According to an exemplary embodiment, in operation 204, the electronic device 101 may receive, from the external electronic device 102, a movement distance change amount of the external electronic device 102 for at least one axis indicating a preset direction, which is acquired through a second inertial sensor of the external electronic device 102.

According to an exemplary embodiment, in operation 204, the electronic device 101 may acquire the first feature value, based on the movement distance change amount of the electronic device 101 and the movement distance change amount of the external electronic device 102. A detailed description of this will be made later.

According to an exemplary embodiment, in operation 204, the electronic device 101 may acquire the second feature value by using the at least one third sensor. The at least one third sensor of an exemplary embodiment may include a UWB antenna. In operation 204, the electronic device 101 may acquire a distance between the electronic device 101 and the external electronic device 102 as the second feature value, by using the UWB antenna.

According to an exemplary embodiment, in operation 205, the electronic device 101 may acquire an eye fatigue level (F) of a user, based on the first feature value and the second feature value acquired through operation 204. An operation in which the electronic device 101 of an exemplary embodiment acquires the eye fatigue level of the user, based on the first feature value and the second feature value in operation 205 may be referred to as equation below.

[Equation 1]

$$F = (\frac{1}{D} \cdot \alpha + M \cdot \beta) \times T$$

According to an exemplary embodiment, the eye fatigue level of the user may increase as the first feature value (M) is larger and the second feature value (D) is smaller. According to an exemplary embodiment, the eye fatigue level of the user may increase as a time (T) for which the user gazes at the display of the electronic device 101 increases.

The first feature value (M) of an exemplary embodiment may be referred to as a difference of a shaking degree between the electronic device 101 and the external electronic device 102. According to an exemplary embodiment, the second feature value (D) may be referred to as the distance between the electronic device 101 and the external electronic device 102. A detailed description of this will be made later.

According to an exemplary embodiment, the user's eye fatigue level (F) may be stored in a memory in the form of a look-up table including a value outputted by Equation 1.

According to an exemplary embodiment, in operation 206, the electronic device 101 may control the display of the electronic device 101, based on the fatigue level (F) acquired through operation 205. According to an exemplary embodiment, in operation 206, the electronic device 101 may control the display of the electronic device 101 in order to reduce the fatigue level (F), based on the fatigue level (F) acquired through operation 205. For example, in operation 206, the electronic device 101 may control the display of the electronic device 101 for a specified time so as to reduce the fatigue level (F). For another example, in operation 206, the electronic device 101 may control the display of the electronic device 101 in real time so as to reduce the fatigue level (F). A detailed description of this will be made later.

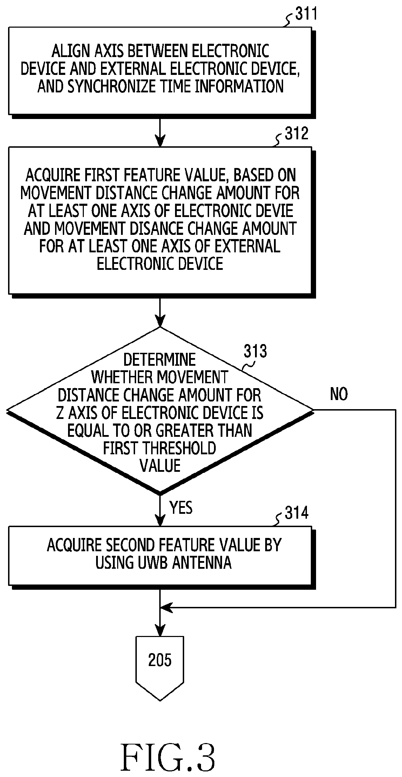

FIG. 3 is a flowchart illustrating an operation of acquiring a first feature value and a second feature value by an electronic device according to an exemplary embodiment.

Referring to FIG. 3, the electronic device 101 of an exemplary embodiment may acquire a first feature value, and acquire a second feature value, based on the first feature value.

According to an exemplary embodiment, in operation 311, the electronic device 101 may align an axis with the external electronic device 102, and synchronize time information.

According to an exemplary embodiment, in operation 311, the electronic device 101 may acquire data on at least one axis within a space where the external electronic device 102 is located from the external electronic device 102. According to an exemplary embodiment, in operation 311, the electronic device 101 may correct at least one axis within a space where the electronic device 101 is located, based on the data acquired from the external electronic device 102. According to an exemplary embodiment, in operation 311, the electronic device 101 may align the axis of the electronic device 101 with the axis of the external electronic device 102, based on the axis data acquired from the external electronic device 102. For example, the electronic device 101 may equally align the axes between the electronic device 101 and the external electronic device 102 through operation 311. For example, the electronic device 101 may equally align the axes between the electronic device 101 and the external electronic devices 102, by rotating and correcting each axis of an inertial sensor through a rotation matrix with a criterion of a position where the electronic device 101 is worn on the user's body.

According to another exemplary embodiment, in operation 311, the electronic device 101 may correct at least one axis of the external electronic device 102, based on data on at least one axis within a space where the electronic device 101 is located.

According to an exemplary embodiment, in operation 311, the electronic device 101 may receive, from the external electronic device 102, time information related to a movement distance change amount for the at least one axis of the external electronic device 102.

According to an exemplary embodiment, the electronic device 101 may correct the movement distance change amount of the external electronic device 102 received from the external electronic device 101, based on the time information of the external electronic device 102 received in operation 311 and a movement distance change amount for at least one axis of the electronic device 101.

An operation of synchronizing the time information of the electronic device 101 and the external electronic device 102 in operation 311 by the electronic device 101 of an exemplary embodiment may be referred to by Equation 2 below.

**【Equation 2】**

$$\mathrm{Xi} = (\mathrm{X}_{i\text{-}1} \pm \Delta \mathrm{w}_x) \times \mathrm{delay}_{latency}$$
$$\mathrm{Yi} = (\mathrm{Y}_{i\text{-}1} \pm \Delta \mathrm{w}_y) \times \mathrm{delay}_{latency}$$
$$\mathrm{Zi} = (\mathrm{Z}_{i\text{-}1} \pm \Delta \mathrm{w}_z) \times \mathrm{delay}_{latency}$$

According to an exemplary embodiment, the electronic device 101 may synchronize the time information between the electronic device 101 and the external electronic device 102, based on the time information of the external electronic device 102 received from the external electronic device 102 and the delay time ($delay_{latency}$) acquired based on the time information. For example, the compensation value ($\Delta w$) dependent on the delay time ($delay_{latency}$) may be previously specified and stored in a memory. For another example, the compensation value ($\Delta w$) dependent on the delay time ($delay_{latency}$) may be determined in real time by a modeled function.

According to an exemplary embodiment, in operation 312, the electronic device 101 may acquire a first feature value by using at least one third sensor.

According to an exemplary embodiment, in operation 312, the electronic device 101 may acquire a movement distance change amount for at least one axis (e.g., z-axis) indicating a preset direction (e.g., a direction perpendicular to the ground) within a space where the electronic device 101 is located, by using the at least one third sensor (or a first inertial sensor).

According to an exemplary embodiment, in operation 312, the electronic device 101 may receive, from the external electronic device 102, a movement distance change amount of the external electronic device 102 for at least one axis indicating a preset direction, which is acquired through a second inertial sensor of the external electronic device 102.

According to an exemplary embodiment, in operation 312, the electronic device 101 may acquire a first feature value, based on the movement distance change amount of the electronic device 101 and the movement distance change amount of the external electronic device 102. According to an exemplary embodiment, in operation 312, the electronic device 101 may acquire the first feature value, based on a difference between the movement distance change amount of the electronic device 101 and the movement distance change amount of the external electronic device 102. For example, the first feature value may be referred to as a difference of a shaking degree between the electronic device 101 and the external electronic device 102.

According to an exemplary embodiment, in operation 313, the electronic device 101 may determine whether a movement distance change amount for z-axis among the movement distance change amount of the electronic device 101 is greater than or equal to a first threshold value. For example, in operation 313, the electronic device 101 may determine whether the electronic device 101 shakes in a direction perpendicular to the ground by a first threshold value or more.

According to an exemplary embodiment, in operation 314, the electronic device 101 may acquire a second feature value by using a UWB antenna. According to an exemplary embodiment, in response to the movement distance change amount for z-axis among the movement distance change amount of the electronic device 101 being greater than or equal to the first threshold value, in operation 314, the electronic device 101 may acquire the second feature value by using the UWB antenna. According to an exemplary embodiment, in response to the movement distance change amount for z-axis among the movement distance change amount of the electronic device 101 being greater than or equal to the first threshold value, in operation 314, the electronic device 101 may acquire the second feature value by driving the UWB antenna.

According to an exemplary embodiment, in operation 314, the electronic device 101 may measure a distance between the electronic device 101 and the external electronic device 102 by using the UWB antenna. For example, the second feature value may be referred to as the distance between the electronic device 101 and the external electronic device 102.

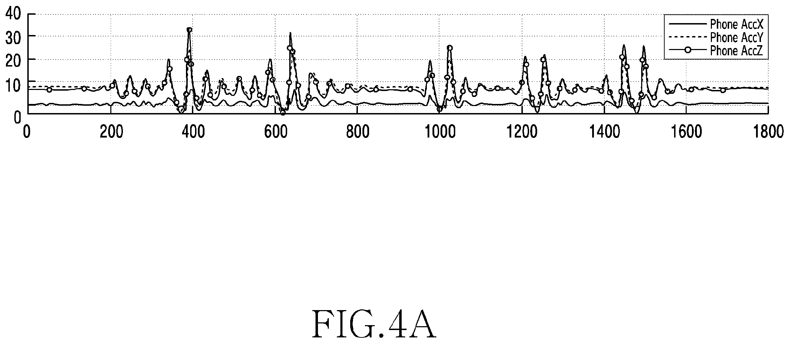

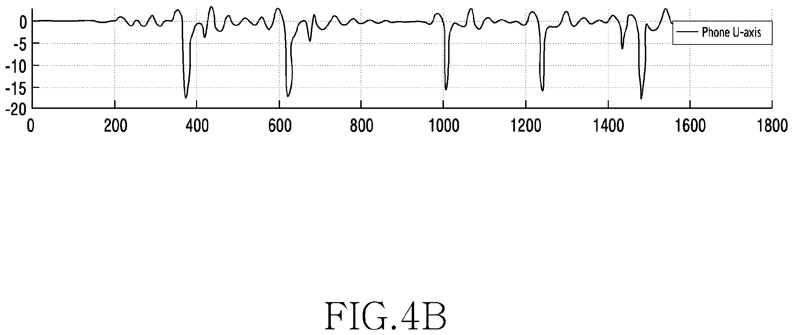

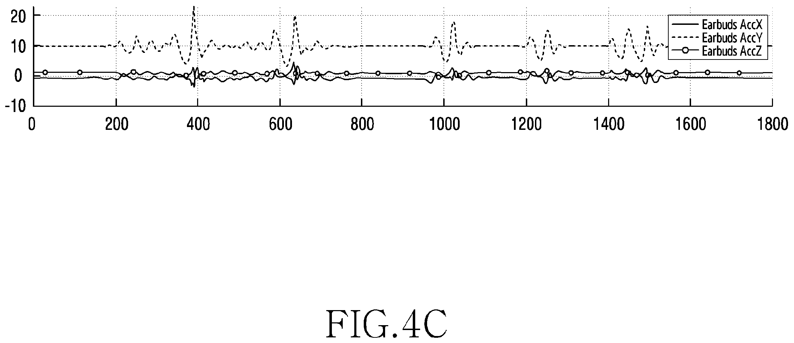

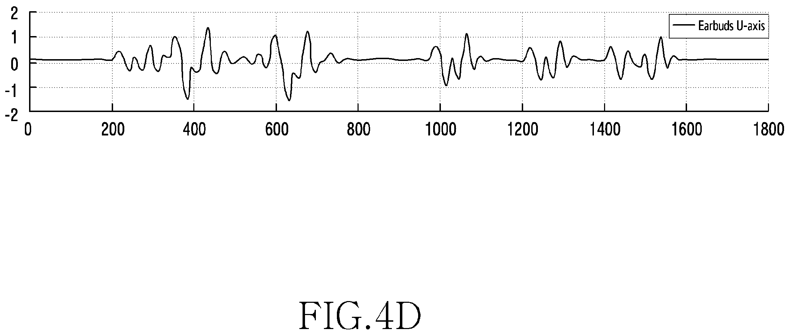

FIG. 4A illustrates a movement distance change amount for each axis of an electronic device according to an exemplary embodiment. FIG. 4B illustrates a movement distance change amount of a direction perpendicular to the ground of the electronic device according to an exemplary embodiment. FIG. 4C illustrates a movement distance change amount for each axis of an external electronic device according to an exemplary embodiment. FIG. 4D illustrates a movement distance change amount of a direction perpendicular to the ground of the external electronic device according to an exemplary embodiment.

Referring to FIG. 4A to FIG. 4D, the electronic device 101 of an exemplary embodiment may have a larger movement distance change amount for at least one axis than the external electronic device 102. FIG. 4A to FIG. 4D of an exemplary embodiment may be referred to as a movement distance change amount for each axis of the electronic device 101 and/or the external electronic device 102 for a specified time. At this time, the movement distance change amount for each axis may be referred to as an acceleration value. It is not limited thereto, and may refer to various parameters capable of indicating the change of a movement distance.

Referring to FIG. 4A and FIG. 4C of an exemplary embodiment, the electronic device 101 of an exemplary embodiment may have a larger movement distance change amount than the external electronic device 102, with respect to each axis within a space in which the electronic device 101 and the external electronic device 102 exist.

Referring to FIG. 4B and FIG. 4D of an exemplary embodiment, the electronic device 101 of an exemplary embodiment may have a larger movement distance change amount than the external electronic device 102, with respect to a z-axis or an axis indicating a direction perpendicular to the ground within the space in which the electronic device 101 and the external electronic device 102 exist.

According to an exemplary embodiment, the electronic device 101 may shake relatively more than the external electronic device 102 due to an external force in a vehicle riding or walking situation.

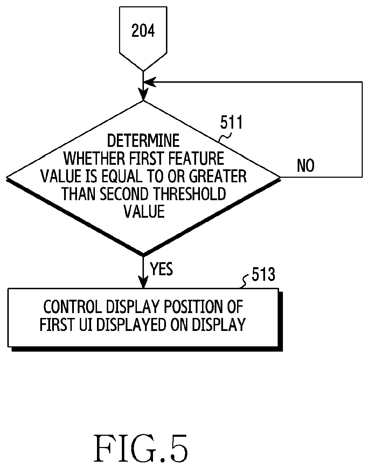

FIG. 5 is a flowchart illustrating an operation of controlling a display position of a first UI displayed on a display by an electronic device according to an exemplary embodiment.

Referring to FIG. 5, the electronic device 101 of an exemplary embodiment may control the display position of the first user interface (UI) displayed on the display, when a first feature value is greater than or equal to a second threshold value.

According to an exemplary embodiment, in operation 511, the electronic device 101 may determine whether the first feature value acquired through operation 204 is greater than or equal to the second threshold value. According to an exemplary embodiment, in operation 511, the electronic device 101 may determine whether a difference of a shaking degree between the electronic device 101 and the external electronic device 102 acquired through operation 204 is greater than or equal to a predetermined second threshold value.

According to an exemplary embodiment, in operation 513, in response to the first feature value being greater than or equal to the second threshold value, the electronic device 101 may control the display position of the first UI displayed on the display. According to an exemplary embodiment, in operation 513, in response to the first feature value being greater than or equal to the second threshold value, the electronic device 101 may control the display position of the first UI displayed on the display in real time.

For example, when the electronic device 101 moves relatively greatly compared to the external electronic device 102 in a +z-axis direction, in operation 513, the electronic device 101 may change a position of the first UI displayed on the display into a -z-axis direction. A detailed description of this will be made later.

For example, the first UI may be referred to as an e-book or a video playback interface, but is not limited thereto.

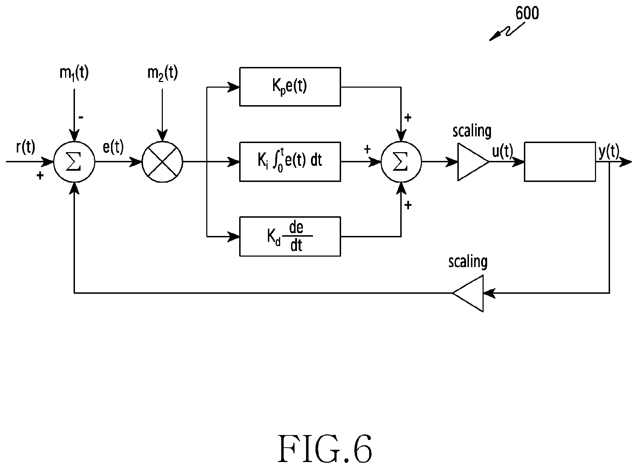

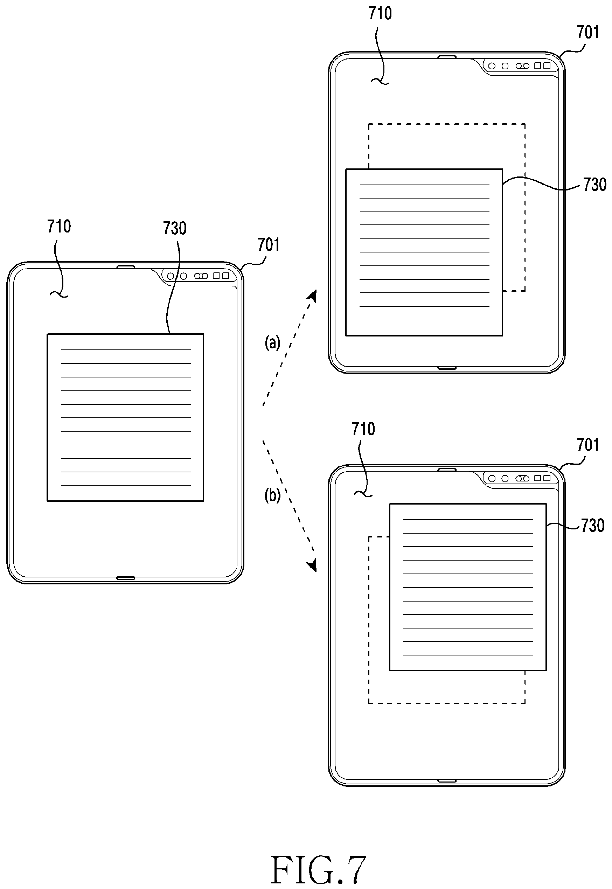

FIG. 6 illustrates an output control model for a UI of an electronic device according to an exemplary embodiment. FIG. 7 illustrates a construction of controlling a display position of a first UI displayed on a display by the electronic device according to an exemplary embodiment.

Referring to FIG. 5 to FIG. 7 together, the electronic device 701 (e.g., the electronic device 101 of FIG. 1) of an exemplary embodiment may control the display position of the first UI 730 displayed on the display 710 through the output control model 600.

Referring to FIG. 6, the electronic device 701 of an exemplary embodiment may change the display position of the first UI 730, based on a first feature value ($m_1$(t)), a second feature value ($m_1$(t)), and a current display position (y(t)) of the first UI 730. According to an exemplary embodiment, the electronic device 701 may input the first feature value ($m_1$(t)), the second feature value ($m_1$(t)), and the current display position (y(t)) of the first UI 730 to the output control model 600, and acquire an output position compensation value (u(t)). According to an exemplary embodiment, the electronic device 701 may change the current display position (y(t)) of the first UI 730, by applying the acquired output position compensation value (u(t)) through the output control model 600.

An operation of acquiring the output position compensation value (u(t)) through the output control model 600 of an exemplary embodiment may be referred by equation below.

【Equation 3】

$$u(t) = K_p e(t) + K_i \int_0^t e(t)dt + K_d \frac{de}{dt}$$

In this case, $K_p$, $K_i$, $K_d$ may be referred to as gain values for proportional, integral, and differential terms, respectively. The gain values for the respective proportional, integral, and differential terms may be stored in a memory.

According to an exemplary embodiment, the electronic device 701 may control the display position of the first UI 730 displayed on the display 710 through the output control model 600 wherein the focus of user's eyes does not shake. According to an exemplary embodiment, the electronic device 701 may control the display position of the first UI 730 displayed on the display 710 through the output control model 600, in order to minimize the shaking of the focus of the user's eyes.

For example, when the electronic device 701 moves relatively greatly in an upper right direction compared to the external electronic device 102 (a), the position of the first UI 730 displayed on the display 710 may be changed in a lower left direction.

For another example, when the electronic device 701 moves relatively greatly in the lower left direction compared to the external electronic device 102 (b), the position of the first **UI** 730 displayed on the display 710 may be changed in the upper right direction.

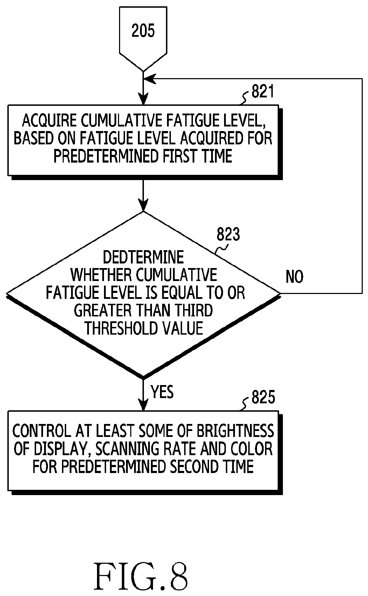

FIG. 8 is a flowchart illustrating an operation of acquiring a cumulative fatigue level for a predetermined time and controlling at least some of a brightness of a display, a scanning rate, and a color, based on the acquired fatigue level, by an electronic device according to an exemplary embodiment.

Referring to FIG. 8, the electronic device 101 of an exemplary embodiment may acquire a cumulative fatigue level accumulated for a specified first time, and control a display (e.g., the display 710 of FIG. 7) for a specified second time, based on the cumulative fatigue level.

According to an exemplary embodiment, in operation 821, the electronic device 101 may acquire a cumulative fatigue level accumulated for a predetermined first time, based on the user's eye fatigue level acquired through operation 205. For example, the predetermined first time may be referred to as 12 hours or 24 hours, but is not limited thereto.

According to an exemplary embodiment, in operation 823, the electronic device 101 may determine whether the cumulative fatigue level acquired through operation 821 is greater than or equal to a third threshold value. For example, in operation 823, the electronic device 101 may determine whether the cumulative fatigue level accumulated for 24 hours is greater than or equal to a predetermined third threshold.

According to an exemplary embodiment, in operation 825, in response to the cumulative fatigue level being equal to or greater than the third threshold value, the electronic device 101 may control at least some of a brightness of the display, a scanning rate, and a color for a predetermined second time.

For example, in operation 825, in response to the cumulative fatigue level being equal to or greater than the third threshold value, the electronic device 101 may reduce the brightness of the display for 12 hours. For another example, in operation 825, in response to the cumulative fatigue level being equal to or greater than the third threshold value, the electronic device 101 may increase the scanning rate of the display for 8 hours. For further example, in operation 825, in response to the cumulative fatigue level being equal to or greater than the third threshold value, the electronic device 101 may control the color of the display to a relatively warm color. According to another example (not shown), in operation 825, in response to the cumulative fatigue level being equal to or greater than the third threshold value, the electronic device 101 may apply a blue light filter to a screen outputted through the display.

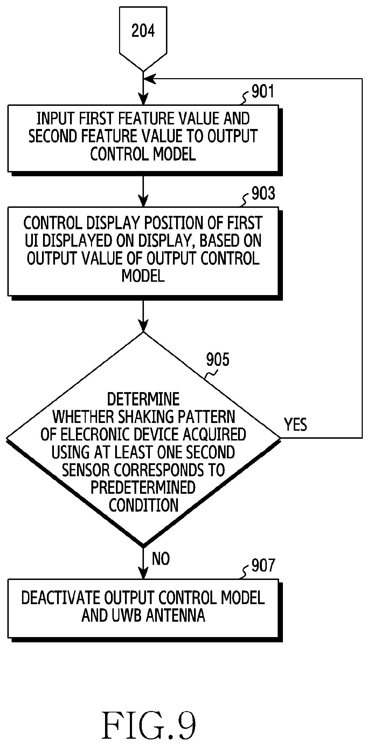

FIG. 9 is a flowchart illustrating an operation of deactivating an output control model and a UWB antenna when a shaking pattern of an electronic device does not correspond to a predetermined condition according to an exemplary embodiment.

Referring to FIG. 9, when the shaking pattern of the electronic device 101 does not correspond to the predetermined condition, the electronic device 101 of an exemplary embodiment may deactivate the output control model 600 and the UWB antenna in order to reduce power consumption.

According to an exemplary embodiment, in operation 901, the electronic device 101 may input, to the output control model 600, a first feature value and a second feature value acquired through operation 204.

According to an exemplary embodiment, in operation 903, the electronic device 101 may control a display position of a first UI (e.g., the first UI 710 of FIG. 7) displayed on a display (e.g., the display 710 of FIG. 7), based on an output value (y(t)) of the output control model 600. Operation 903 of an exemplary embodiment may be referred to as operation 513 of FIG. 5.

According to an exemplary embodiment, in operation 905, the electronic device 101 may acquire a shaking pattern of the electronic device 101 by using at least one second sensor. According to an exemplary embodiment, in operation 905, the electronic device 101 may determine whether the shaking pattern acquired using the at least one second sensor corresponds to a predetermined condition. For example, in operation 905, the electronic device 101 may determine whether the shaking pattern acquired using the at least one second sensor corresponds to a shaking pattern of a situation where a user gets in a vehicle. For another example, in operation 905, the electronic device 101 may determine whether the shaking pattern acquired using the at least one second sensor corresponds to a shaking pattern of a situation where the user is walking. Operation 905 of an exemplary embodiment may be referred to as operation 203.

According to an exemplary embodiment, in operation 907, the electronic device 101 may deactivate the output control model 600 and the UWB antenna. According to an exemplary embodiment, in operation 907, in response to the shaking pattern of the electronic device 101 not corresponding to the predetermined condition, the electronic device 101 may deactivate the output control model 600 and the UWB antenna. According to an exemplary embodiment, in operation 907, in response to the shaking pattern of the electronic device 101 not corresponding to the predetermined condition, the electronic device 101 may deactivate the output control model 600 and the UWB antenna in order to reduce power consumption.

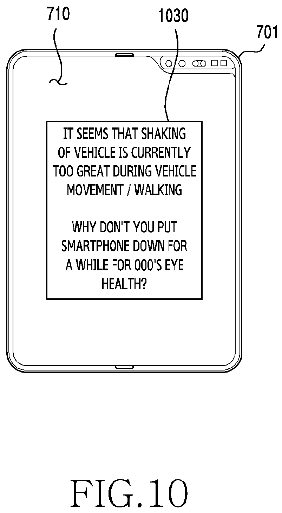

FIG. 10 illustrates a construction of displaying a second UI on a display as a display position of a first UI is changed according to an exemplary embodiment.

Referring to FIG. 7 and FIG. 10 together, when a distance between a current display position of the first UI 730 and a position to display the first UI 730 is equal to or greater than a predetermined threshold value, the electronic device 701 of an exemplary embodiment may display a second UI 1030 different from the first UI 730 through the display 710.

According to another exemplary embodiment, when a first feature value is greater than or equal to a predetermined threshold value, the electronic device 701 may display the second UI 1030 different from the first UI 730 through the display 710.

According to an exemplary embodiment, the second UI 1030 may include guide information for reducing an eye fatigue level of a user. According to an exemplary embodiment, the second UI 1030 may include a message of recommending the user to stop using the electronic device 101. The second UI 1030 may include a message of recommending the user to stop gazing at the electronic device 101.

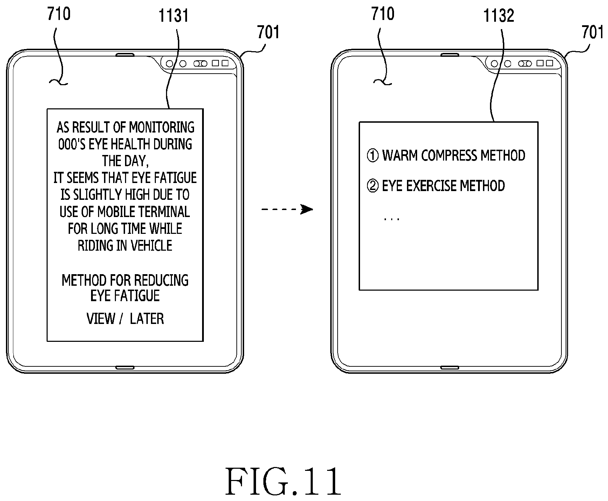

FIG. 11 illustrates a construction of displaying a third UI capable of receiving a user's input through a display, based on a cumulative fatigue level according to an exemplary embodiment.

Referring to FIG. 11, the electronic device 701 of an exemplary embodiment may display a 3-1st UI 1131 capable of receiving a user's input through the display 710, based on a cumulative fatigue level, for a specified time.

According to an exemplary embodiment, the electronic device 701 may acquire a cumulative fatigue level accumulated for a predetermined first time, based on a user's eye fatigue level.

According to an exemplary embodiment, when the acquired cumulative fatigue level exceeds a specified threshold value, the electronic device 701 may display the 3-1st UI 1131 capable of receiving the user's input through the display 710. For example, when the cumulative fatigue level acquired by the electronic device 701 for 24 hours exceeds the specified threshold value, the electronic device 701 may display the 3-1st UI 1131 including information related to the cumulative fatigue level, through the display 710.

According to an exemplary embodiment, the electronic device 701 may display, through the display 710, a 3-2nd UI 1132 including guide information for reducing the user's eye fatigue level, based on the user's input to the 3-1st UI 1131.

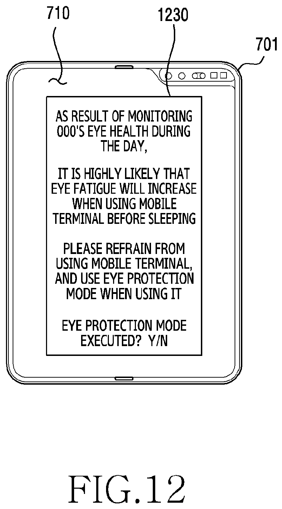

FIG. 12 illustrates a construction of displaying a fourth UI through a display at a specified time, based on a cumulative fatigue level according to an exemplary embodiment.

Referring to FIG. 12, the electronic device 701 of an exemplary embodiment may display the fourth UI 1230 capable of receiving a user's input through the display 710, based on a cumulative fatigue level, for a specified time.

According to an exemplary embodiment, when the cumulative fatigue level exceeds a predetermined threshold value for a specified time, the electronic device 701 may display the fourth UI 1230 capable of receiving the user's input through the display 710. For example, when the cumulative fatigue level acquired by the electronic device 701 for 24 hours exceeds a specified threshold value, the electronic device 701 may display the fourth UI 1230 including information related to the cumulative fatigue level through the display 710.

According to an exemplary embodiment, when the cumulative fatigue level accumulated for a specified time exceeds a predetermined threshold value, the electronic device 101 may display the fourth UI 1230 capable of receiving a user's input through the display 710. For example, when the cumulative fatigue level accumulated over the past 24 hours at midnight every day exceeds a predetermined threshold value, the electronic device 101 may display the fourth UI 1230 capable of receiving a user's input through the display 710. For another example, when the cumulative fatigue level accumulated over the past 12 hours at noon every day exceeds a predetermined threshold value, the electronic device 101 may display the fourth UI 1230 capable of receiving a user's input through the display 710.

According to an exemplary embodiment, in response to the user's input to the fourth UI 1230, the electronic device 701 may execute an eye protection mode. According to an exemplary embodiment, in response to the user's input to the fourth UI 1230, the electronic device 701 may lower a brightness of the display 710, increase a scanning rate, or change a color into a relatively warm color. According to another exemplary embodiment, in response to the user's input to the fourth UI 1230, the electronic device 701 may execute a blue light filter. According to an exemplary embodiment, an operation of executing the eye protection mode by the electronic device 701 in response to the user's input to the fourth UI 1230 may be referred to as operation 825 of FIG. 8.

FIG. 13 is a block diagram illustrating an electronic device 101 in a network environment 1300 according to various embodiments.

Referring to FIG. 13, the electronic device 1301 in the network environment 1300 may communicate with an electronic device 1302 via a first network 1398 (e.g., a short-range wireless communication network), or at least one of an electronic device 1304 or a server 1308 via a second network 1399 (e.g., a long-range wireless communication network). According to an exemplary embodiment, the electronic device 1301 may communicate with the electronic device 1304 via the server 1308. According to an exemplary embodiment, the electronic device 1301 may include a processor 1320, memory 130, an input module 1350, a sound output module 1355, a display module 1360, an audio module 1370, a sensor module 1376, an interface 1377, a connecting terminal 1378, a haptic module 1379, a camera module 1380, a power management module 1388, a battery 1389, a communication module 1390, a subscriber identification module(SIM) 1396, or an antenna module 1397. In some embodiments, at least one of the components (e.g., the connecting terminal 1378) may be omitted from the electronic device 1301, or one or more other components may be added in the electronic device 1301. In some embodiments, some of the components (e.g., the sensor module 1376, the camera module 1380, or the antenna module 1397) may be implemented as a single component (e.g., the display module 1360).

The processor 1320 may execute, for example, software (e.g., a program 1340) to control at least one other component (e.g., a hardware or software component) of the electronic device 1301 coupled with the processor 1320, and may perform various data processing or computation. According to one embodiment, as at least part of the data processing or computation, the processor 1320 may store a command or data received from another component (e.g., the sensor module 1376 or the communication module 1390) in volatile memory 1332, process the command or the data stored in the volatile memory 1332, and store resulting data in non-volatile memory 1334. According to an exemplary embodiment, the processor 1320 may include a main processor 1321 (e.g., a central processing unit (CPU) or an application processor (AP)), or an auxiliary processor 1323 (e.g., a graphics processing unit (GPU), a neural processing unit (NPU), an image signal processor (ISP), a sensor hub processor, or a communication processor (CP)) that is operable independently from, or in conjunction with, the main processor 1321. For example, when the electronic device 1301 includes the main processor 1321 and the auxiliary processor 1323, the auxiliary processor 1323 may be adapted to consume less power than the main processor 1321, or to be specific to a specified function. The auxiliary processor 1323 may be implemented as separate from, or as part of the main processor 1321.

The auxiliary processor 1323 may control at least some of functions or states related to at least one component (e.g., the display module 1360, the sensor module 1376, or the communication module 1390) among the components of the electronic device 1301, instead of the main processor 1321 while the main processor 1321 is in an inactive (e.g., sleep) state, or together with the main processor 1321 while the main processor 1321 is in an active state (e.g., executing an application). According to an exemplary embodiment, the auxiliary processor 1323 (e.g., an image signal processor or a communication processor) may be implemented as part of another component (e.g., the camera module 1380 or the communication module 1390) functionally related to the auxiliary processor 1323. According to an exemplary embodiment, the auxiliary processor 1323 (e.g., the neural processing unit) may include a hardware structure specified for artificial intelligence model processing. An artificial intelligence model may be generated by machine learning. Such learning may be performed, e.g., by the electronic device 1301 where the artificial intelligence is performed or via a separate server (e.g., the server 1308). Learning algorithms may include, but are not limited to, e.g., supervised learning, unsupervised learning, semi-supervised learning, or reinforcement learning. The artificial intelligence model may include a plurality of artificial neural network layers. The artificial neural network may be a deep neural network (DNN), a convolutional neural network (CNN), a recurrent neural network (RNN), a restricted boltzmann machine (RBM), a deep belief network (DBN), a bidirectional recurrent deep neural network (BRDNN), deep Q-network or a combination of two or more thereof but is not limited thereto. The artificial intelligence model may, additionally or alternatively, include a software structure other than the hardware structure.

The memory 1330 may store various data used by at least one component (e.g., the processor 1320 or the sensor module 1376) of the electronic device 1301. The various data may include, for example, software (e.g., the program 1340) and input data or output data for a command related thererto. The memory 1330 may include the volatile memory 1332 or the non-volatile memory 1334.

The program 1340 may be stored in the memory 1330 as software, and may include, for example, an operating system (OS) 1342, middleware 1344, or an application 1346.

The input module 1350 may receive a command or data to be used by another component (e.g., the processor 1320) of the electronic device 1301, from the outside (e.g., a user) of the electronic device 1301. The input module 1350 may include, for example, a microphone, a mouse, a keyboard, a key (e.g., a button), or a digital pen (e.g., a stylus pen).

The sound output module 1355 may output sound signals to the outside of the electronic device 1301. The sound output module 1355 may include, for example, a speaker or a receiver. The speaker may be used for general purposes, such as playing multimedia or playing record. The receiver may be used for receiving incoming calls. According to an exemplary embodiment, the receiver may be implemented as separate from, or as part of the speaker.

The display module 1360 may visually provide information to the outside (e.g., a user) of the electronic device 1301. The display module 1360 may include, for example, a display, a hologram device, or a projector and control circuitry to control a corresponding one of the display, hologram device, and projector. According to an exemplary embodiment, the display module 1360 may include a touch sensor adapted to detect a touch, or a pressure sensor adapted to measure the intensity of force incurred by the touch.

The audio module 1370 may convert a sound into an electrical signal and vice versa. According to an exemplary embodiment, the audio module 1370 may obtain the sound via the input module 1350, or output the sound via the sound output module 1355 or a headphone of an external electronic device (e.g., an electronic device 1302) directly (e.g., wiredly) or wirelessly coupled with the electronic device 1301.

The sensor module 1376 may detect an operational state (e.g., power or temperature) of the electronic device 1301 or an environmental state (e.g., a state of a user) external to the electronic device 1301, and then generate an electrical signal or data value corresponding to the detected state. According to an exemplary embodiment, the sensor module 1376 may include, for example, a gesture sensor, a gyro sensor, an atmospheric pressure sensor, a magnetic sensor, an acceleration sensor, a grip sensor, a proximity sensor, a color sensor, an infrared (IR) sensor, a biometric sensor, a temperature sensor, a humidity sensor, or an illuminance sensor.

The interface 1377 may support one or more specified protocols to be used for the electronic device 1301 to be coupled with the external electronic device (e.g., the electronic device 1302) directly (e.g., wiredly) or wirelessly. According to an exemplary embodiment, the interface 1377 may include, for example, a high definition multimedia interface (HDMI), a universal serial bus (USB) interface, a secure digital (SD) card interface, or an audio interface.

The connecting terminal 1378 may include a connector via which the electronic device 1301 may be physically connected with the external electronic device (e.g., the electronic device 1302). According to an exemplary embodiment, the connecting terminal 1378 may include, for example, a HDMI connector, a USB connector, a SD card connector, or an audio connector (e.g., a headphone connector).

The haptic module 1379 may convert an electrical signal into a mechanical stimulus (e.g., a vibration or a movement) or electrical stimulus which may be recognized by a user via his tactile sensation or kinesthetic sensation. According to an exemplary embodiment, the haptic module 1379 may include, for example, a motor, a piezoelectric element, or an electric stimulator.

The camera module 1380 may capture a still image or moving images. According to an exemplary embodiment, the camera module 1380 may include one or more lenses, image sensors, image signal processors, or flashes.

The power management module 1388 may manage power supplied to the electronic device 1301. According to one embodiment, the power management module 1388 may be implemented as at least part of, for example, a power management integrated circuit (PMIC).

The battery 1389 may supply power to at least one component of the electronic device 1301. According to an exemplary embodiment, the battery 1389 may include, for example, a primary cell which is not rechargeable, a secondary cell which is rechargeable, or a fuel cell.

The communication module 1390 may support establishing a direct (e.g., wired) communication channel or a wireless communication channel between the electronic device 1301 and the external electronic device (e.g., the electronic device 1302, the electronic device 1304, or the server 1308) and performing communication via the established communication channel. The communication module 1390 may include one or more communication processors that are operable independently from the processor 1320 (e.g., the application processor (AP)) and supports a direct (e.g., wired) communication or a wireless communication. According to an exemplary embodiment, the communication module 1390 may include a wireless communication module 1392 (e.g., a cellular communication module, a short-range wireless communication module, or a global navigation satellite system (GNSS) communication module) or a wired communication module 1394 (e.g., a local area network (LAN) communication module or a power line communication (PLC) module). A corresponding one of these communication modules may communicate with the external electronic device via the first network 1398 (e.g., a short-range communication network, such as Bluetooth™, wireless-fidelity (Wi-Fi) direct, or infrared data association (IrDA)) or the second network 1399 (e.g., a long-range communication network, such as a legacy cellular network, a 5G network, a next-generation communication network, the Internet, or a computer network (e.g., LAN or wide area network (WAN)). These various types of communication modules may be implemented as a single component (e.g., a single chip), or may be implemented as multi components (e.g., multi chips) separate from each other. The wireless communication module 1392 may identify and authenticate the electronic device 1301 in a communication network, such as the first network 1398 or the second network 1399, using subscriber information (e.g., international mobile subscriber identity (IMSI)) stored in the subscriber identification module 1396.

The wireless communication module 1392 may support a 5G network, after a 4G network, and next-generation communication technology, e.g., new radio (NR) access technology. The NR access technology may support enhanced mobile broadband (eMBB), massive machine type communications (mMTC), or ultra-reliable and low-latency communications (URLLC). The wireless communication module 1392 may support a high-frequency band (e.g., the mmWave band) to achieve, e.g., a high data transmission rate. The wireless communication module 1392 may support various technologies for securing performance on a high-frequency band, such as, e.g., beamforming, massive multiple-input and multiple-output (massive MIMO), full dimensional MIMO (FD-MIMO), array antenna, analog beam-forming, or large scale antenna. The wireless communication module 1392 may support various requirements specified in the electronic device 1301, an external electronic device (e.g., the electronic device 1304), or a network system (e.g., the second network 1399). According to an exemplary embodiment, the wireless communication module 1392 may support a peak data rate (e.g., 20Gbps or more) for implementing eMBB, loss coverage (e.g., 164dB or less) for implementing mMTC, or U-plane latency (e.g., 0.5ms or less for each of downlink (DL) and uplink (UL), or a round trip of 1ms or less) for implementing URLLC.

The antenna module 1397 may transmit or receive a signal or power to or from the outside (e.g., the external electronic device) of the electronic device 1301. According to an exemplary embodiment, the antenna module 1397 may include an antenna including a radiating element composed of a conductive material or a conductive pattern formed in or on a substrate (e.g., a printed circuit board (PCB)). According to an exemplary embodiment, the antenna module 1397 may include a plurality of antennas (e.g., array antennas). In such a case, at least one antenna appropriate for a communication scheme used in the communication network, such as the first network 1398 or the second network 1399, may be selected, for example, by the communication module 1390 (e.g., the wireless communication module 1392) from the plurality of antennas. The signal or the power may then be transmitted or received between the communication module 1390 and the external electronic device via the selected at least one antenna. According to an exemplary embodiment, another component (e.g., a radio frequency integrated circuit (RFIC)) other than the radiating element may be additionally formed as part of the antenna module 1397.

According to various embodiments, the antenna module 1397 may form a mmWave antenna module. According to an exemplary embodiment, the mmWave antenna module may include a printed circuit board, a RFIC disposed on a first surface (e.g., the bottom surface) of the printed circuit board, or adjacent to the first surface and capable of supporting a designated high-frequency band (e.g., the mmWave band), and a plurality of antennas (e.g., array antennas) disposed on a second surface (e.g., the top or a side surface) of the printed circuit board, or adjacent to the second surface and capable of transmitting or receiving signals of the designated high-frequency band.

At least some of the above-described components may be coupled mutually and communicate signals (e.g., commands or data) therebetween via an inter-peripheral communication scheme (e.g., a bus, general purpose input and output (GPIO), serial peripheral interface (SPI), or mobile industry processor interface (MIPI)).

According to an exemplary embodiment, commands or data may be transmitted or received between the electronic device 1301 and the external electronic device 1304 via the server 1308 coupled with the second network 1399. Each of the electronic devices 1302 or 1304 may be a device of a same type as, or a different type, from the electronic device 1301. According to an exemplary embodiment, all or some of operations to be executed at the electronic device 1301 may be executed at one or more of the external electronic devices 1302, 1304, or 1308. For example, if the electronic device 1301 should perform a function or a service automatically, or in response to a request from a user or another device, the electronic device 1301, instead of, or in addition to, executing the function or the service, may request the one or more external electronic devices to perform at least part of the function or the service. The one or more external electronic devices receiving the request may perform the at least part of the function or the service requested, or an additional function or an additional service related to the request, and transfer an outcome of the performing to the electronic device 1301. The electronic device 1301 may provide the outcome, with or without further processing of the outcome, as at least part of a reply to the request. To that end, a cloud computing, distributed computing, mobile edge computing (MEC), or client-server computing technology may be used, for example. The electronic device 1301 may provide ultra low-latency services using, e.g., distributed computing or mobile edge computing. In another exemplary embodiment, the external electronic device 1304 may include an internet-of-things (IoT) device. The server 1308 may be an intelligent server using machine learning and/or a neural network. According to an exemplary embodiment, the external electronic device 1304 or the server 1308 may be included in the second network 1399. The electronic device 1301 may be applied to intelligent services (e.g., smart home, smart city, smart car, or healthcare) based on 5G communication technology or IoT-related technology.

The electronic device according to various embodiments may be one of various types of electronic devices. The electronic devices may include, for example, a portable communication device (e.g., a smartphone), a computer device, a portable multimedia device, a portable medical device, a camera, a wearable device, or a home appliance. According to an exemplary embodiment of the disclosure, the electronic devices are not limited to those described above.

It should be appreciated that various embodiments of the present disclosure and the terms used therein are not intended to limit the technological features set forth herein to particular embodiments and include also other embodiments falling within the scope of the appended claims. With regard to the description of the drawings, similar reference numerals may be used to refer to similar or related elements. It is to be understood that a singular form of a noun corresponding to an item may include one or more of the things, unless the relevant context clearly indicates otherwise. As used herein, each of such phrases as "A or B," "at least one of A and B," "at least one of A or B," "A, B, or C," "at least one of A, B, and C," and "at least one of A, B, or C," may include any one of, or all possible combinations of the items enumerated together in a corresponding one of the phrases. As used herein, such terms as "1st" and "2nd," or "first" and "second" may be used to simply distinguish a corresponding component from another, and does not limit the components in other aspect (e.g., importance or order). It is to be understood that if an element (e.g., a first element) is referred to, with or without the term "operatively" or "communicatively", as "coupled with," "coupled to," "connected with," or "connected to" another element (e.g., a second element), it means that the element may be coupled with the other element directly (e.g., wiredly), wirelessly, or via a third element.

As used in connection with various embodiments of the disclosure, the term "module" may include a unit implemented in hardware, software, or firmware, and may interchangeably be used with other terms, for example, "logic," "logic block," "part," or "circuitry". A module may be a single integral component, or a minimum unit or part thereof, adapted to perform one or more functions. For example, according to an exemplary embodiment, the module may be implemented in a form of an application-specific integrated circuit (ASIC).

Various embodiments as set forth herein may be implemented as software (e.g., the program 1340) including one or more instructions that are stored in a storage medium (e.g., internal memory 1336 or external memory 1338) that is readable by a machine (e.g., the electronic device 1301). For example, a processor (e.g., the processor 1320) of the machine (e.g., the electronic device 1301) may invoke at least one of the one or more instructions stored in the storage medium, and execute it, with or without using one or more other components under the control of the processor. This allows the machine to be operated to perform at least one function according to the at least one instruction invoked. The one or more instructions may include a code generated by a complier or a code executable by an interpreter. The machine-readable storage medium may be provided in the form of a non-transitory storage medium. Wherein, the term "non-transitory" simply means that the storage medium is a tangible device, and does not include a signal (e.g., an electromagnetic wave), but this term does not differentiate between where data is semi-permanently stored in the storage medium and where the data is temporarily stored in the storage medium.

According to an exemplary embodiment, a method according to various embodiments of the disclosure may be included and provided in a computer program product. The computer program product may be traded as a product between a seller and a buyer. The computer program product may be distributed in the form of a machine-readable storage medium (e.g., compact disc read only memory (CD-ROM)), or be distributed (e.g., downloaded or uploaded) online via an application store (e.g., PlayStore™), or between two user devices (e.g., smart phones) directly. If distributed online, at least part of the computer program product may be temporarily generated or at least temporarily stored in the machine-readable storage medium, such as memory of the manufacturer's server, a server of the application store, or a relay server.

According to various embodiments, each component (e.g., a module or a program) of the above-described components may include a single entity or multiple entities, and some of the multiple entities may be separately disposed in different components. According to various embodiments, one or more of the above-described components may be omitted, or one or more other components may be added. Alternatively or additionally, a plurality of components (e.g., modules or programs) may be integrated into a single component. In such a case, according to various embodiments, the integrated component may still perform one or more functions of each of the plurality of components in the same or similar manner as they are performed by a corresponding one of the plurality of components before the integration. According to various embodiments, operations performed by the module, the program, or another component may be carried out sequentially, in parallel, repeatedly, or heuristically, or one or more of the operations may be executed in a different order or omitted, or one or more other operations may be added.

A control method of the electronic device 101 of an exemplary embodiment may include the operations of connecting with an external electronic device 102 worn by a user by using wireless communication, determining whether the user is gazing at the electronic device 101 by using at least one first sensor, acquiring a shaking pattern of the electronic device 101 by using at least one second sensor in response to the user being gazing at the electronic device 101, acquiring a first feature value and a second feature value which are related to a change of position between the electronic device 101 and the external electronic device 102, by using at least one third sensor when the acquired shaking pattern corresponds to a predetermined condition, acquiring an eye fatigue level of the user, based on the first feature value and the second feature value, and controlling a display 710 of the electronic device 101, based on the acquired fatigue level.

According to an exemplary embodiment, the at least one first sensor may include a grip sensor and a motion sensor, and the operation of determining whether the user is using the electronic device 101 may include the operations of determining whether the user is holding the electronic device 101 by using the grip sensor, and determining whether the display 710 of the electronic device 101 faces a user's face by using the motion sensor.

According to an exemplary embodiment, the method may include the operations of detecting a distance to the external electronic device 102 by using a UWB antenna in response to the user being holding the electronic device 101, and the display 710 facing the user's face, detecting an eye area of the user by using a camera, and determining that the user gazes at the electronic device 101 when the distance to the external electronic device 102 is within a specified distance, and the user's eye area is detected.

According to an exemplary embodiment, the at least one second sensor may include an acceleration sensor or a gyro sensor, and the operation of determining whether the determined shaking pattern corresponds to the predetermined condition may include the operation of determining whether the shaking pattern of the electronic device 101 acquired through the acceleration sensor or the gyro sensor corresponds to a shaking pattern of a situation in which the user gets in a vehicle or a shaking pattern of a situation in which the user is walking.

According to an exemplary embodiment, the at least one third sensor may include a first inertial sensor, and the control method of the electronic device 101 may include the operation of acquiring a movement distance change amount for at least one axis indicating a preset direction within a space where the electronic device 101 is located, by using the first inertial sensor.

According to an exemplary embodiment, the control method of the electronic device 101 may include the operations of receiving a movement distance change amount of the external electronic device 102 for the at least one axis indicating the preset direction, which is acquired through a second inertial sensor of the external electronic device 102, from the external electronic device 102 by using the wireless communication, and acquiring the first feature value, based on a difference between the movement distance change amount of the electronic device 101 and the movement distance change amount of the external electronic device 102.

According to an exemplary embodiment, the at least one third sensor may include a UWG antenna, and the second feature value may be referred to as a distance between the electronic device 101 and the external electronic device 102, acquired using the UWB antenna.

According to an exemplary embodiment, the control method of the electronic device 101 may further include the operations of receiving time information related to the movement distance change amount of the external electronic device 102 from the external electronic device 102 by using the wireless communication, and correcting the received movement distance change amount of the external electronic device 102, based on the received time information of the external electronic device 102 and the movement distance change amount of the electronic device 101.

According to an exemplary embodiment, the control method of the electronic device 101 may further include the operations of acquiring data on at least one axis within a space where the external electronic device 102 is located, from the external electronic device 102 by using the wireless communication, and correcting an axis corresponding to the at least one axis in a space where the electronic device 101 is located, based on the acquired data.

According to an exemplary embodiment, the operation of acquiring the first feature value and the second feature value may include the operations of acquiring the first feature value, and acquiring the second feature value in response to the first feature value exceeding a predetermined threshold value.

According to an exemplary embodiment, the operation of controlling the display 710, based on the fatigue level, may include the operation of changing a position of a first user interface (UI) displayed on the display 710, based on the first feature value, a current display position of the first UI, and the second feature value.

According to an exemplary embodiment, the operation of controlling the display 710, based on the fatigue level, may include the operation of displaying a second UI different from the first UI when a distance between the current display position of the first UI and a position to display 710 the first UI is equal to or greater than a predetermined threshold value, and the second UI may include guide information for reducing the eye fatigue level of the user.

According to an exemplary embodiment, the operation of controlling the display 710 may include the operation of controlling at least one of a brightness of the display 710, a scanning rate, and a color.

According to an exemplary embodiment, the operation of controlling the display 710 may include the operations of acquiring a cumulative fatigue level accumulated for a predetermined first time, based on the acquired fatigue level, and controlling at least one of a brightness of the display 710, a scanning rate, and a color for a predetermined second time when the cumulative fatigue level exceeds a predetermined threshold value.

According to an exemplary embodiment, the operation of controlling the display 710 may include the operation of displaying a third UI capable of receiving a user's input at a predetermined time, based on the cumulative fatigue level.

The electronic device 101 of an exemplary embodiment may include a display 710, a wireless communication circuit, and at least one processor electrically connected to the wireless communication circuit. The at least one processor may operatively connect with an external electronic device 102 worn by a user by using the wireless communication circuit, determine whether the user is gazing at the display 710 of the electronic device 101 by using at least one first sensor, acquire a shaking pattern of the electronic device 101 by using at least one second sensor in response to the user being gazing at the electronic device 101, acquire a first feature value and a second feature value which are related to a change of position between the electronic device 101 and the external electronic device 102 by using at least one third sensor when the acquired shaking pattern corresponds to a predetermined condition, acquire an eye fatigue level of the user, based on the first feature value and the second feature value, and control the display 710, based on the acquired fatigue level.

According to an exemplary embodiment, the at least one processor may determine whether the user is holding the electronic device 101 by using a grip sensor, determine whether the display 710 is facing a user's face by using a motion sensor, detect a distance to the external electronic device 102 by using a UWB antenna in response to the user being holding the electronic device 101, and the electronic device 101 facing the user's face, detect a user's eye area by using a camera, determine that the user gazes at the electronic device 101 when a distance to the external electronic device 102 is within a specified distance, and the user's eye area is detected.

According to an exemplary embodiment, the at least one third sensor may include a first inertial sensor, and the at least one processor may acquire a movement distance change amount for at least one axis indicating a preset direction within a space where the electronic device 101 is located, by using the first inertial sensor, receive a movement distance change amount of the external electronic device 102 for the at least one axis indicating the preset direction, which is acquired through a second inertial sensor included in the external electronic device 102, from the external electronic device 102 through the wireless communication circuit, and acquire the first feature value, based on a difference between the movement distance change amount of the electronic device 101 and the movement distance change amount of the external electronic device 102.

According to an exemplary embodiment, the at least one third sensor may include a UWB antenna, and the second feature value may be referred to as a distance between the electronic device 101 and the external electronic device 102, which is acquired using the UWB antenna.

According to an exemplary embodiment, the at least one processor may control at least one of a brightness of the display 710, a color, a scanning rate, and a display position of a UI displayed on the display 710, based on the acquired eye fatigue level of the user.

## Claims

1. An electronic device (101) for reducing an eye fatigue level of a user comprising:

   a display (710);
   a wireless communication circuit; and
   at least one processor (1320),
   memory storing instructions that, when executed by the at least one processor (1320), cause the electronic device (101) to:

   operatively connect (201) with an external electronic device (102) worn by the user by using the wireless communication circuit;
   determine (202) whether the user is gazing at the display (710) of the electronic device (101) by using at least one first sensor;
   in response to the user gazing at the electronic device (101), acquire a shaking pattern of the electronic device (101) by using at least one second sensor;
   when the acquired shaking pattern corresponds to a predetermined condition, acquire (204) a first feature value and a second feature value which are related to a change of position between the electronic device (101) and the external electronic device (102) by using at least one third sensor;
   determine (205) an eye fatigue level of the user, based on the first feature value and the second feature value; and
   control (206) the display (710), based on the determined fatigue level, to reduce the eye fatigue level of the user.

2. The electronic device (101) of claim 1, wherein the instructions, when executed by the at least one processor (1320), cause the electronic device (101) to:

   determine whether the user is holding the electronic device (101) by using a grip sensor;
   determine whether the display (710) is facing a user's face by using a motion sensor;
   in response to the user holding the electronic device (101), and the electronic device (101) facing the user's face, determine a distance to the external electronic device (102) by using an Ultra Wideband, UWB, antenna;
   detect a user's eye area by using a camera;
   when the distance to the external electronic device (102) is within a specified distance, and the user's eye area is detected, determine that the user gazes at the electronic device (101).

3. The electronic device (101) according to any one of the previous claims 1-2, wherein the at least one third sensor comprises a first inertial sensor, and wherein the instructions, when executed by the at least one processor (1320), cause the electronic device (101) to:

   acquire a movement distance change amount for at least one axis representative for a preset direction within a space where the electronic device (101) is located, by using the first inertial sensor;
   receive a movement distance change amount of the external electronic device (102) for the at least one axis representative for the preset direction, which is acquired through a second inertial sensor comprised in the external electronic device (102), from the external electronic device (102) through the wireless communication circuit; and
   acquire the first feature value, based on a difference between the movement distance change amount of the electronic device (101) and the movement distance change amount of the external electronic device (102).

4. The electronic device (101) according to any one of the previous claims 1-3, wherein the second feature value is a distance between the electronic device (101) and the external electronic device (102), which is acquired using an UWB antenna, wherein preferably the at least one third sensor comprises the UWB antenna.

5. The electronic device (101) according to any one of the previous claims 1-4, wherein the instructions, when executed by the at least one processor (1320), cause the electronic device (101) to control at least one of a brightness of the display, a color, a scanning rate, and a display position of a UI displayed on the display, based on the determined eye fatigue level of the user.

6. A control method of an electronic device (101) for reducing an eye fatigue level of a user, the method comprising:

   connecting with an external electronic device (102) worn by the user by using wireless communication;
   determining whether the user is gazing at the electronic device (101) by using at least one first sensor;
   in response to the user gazing at the electronic device (101), acquiring a shaking pattern of the electronic device by using at least one second sensor;
   when the acquired shaking pattern corresponds to a predetermined condition, acquiring a first feature value and a second feature value which are related to a change of position between the electronic device (101) and the external electronic device (102), by using at least one third sensor;

determining an eye fatigue level of the user, based on the first feature value and the second feature value; and

controlling a display (710) of the electronic device (101), based on the determined fatigue level, to reduce the eye fatigue level of the user.

7. The method of claim 6, wherein the at least one first sensor comprises a grip sensor and a motion sensor, and

wherein the step of determining whether the user is gazing the electronic device (101) comprises:

determining whether the user is holding the electronic device (101) by using the grip sensor; and

determining whether the display (710) of the electronic device (101) faces a user's face by using the motion sensor.

8. The method of claim 7, further comprising:

in response to the user being holding the electronic device (101), and the display (710) facing the user's face,

determining a distance to the external electronic device (102) by using an UWB antenna;

detecting an eye area of the user by using a camera; and

when the distance to the external electronic device (102 is within a specified distance, and the user's eye area is detected, determining that the user gazes at the electronic device (101).

9. The method according to any one of the previous claims 6-8, wherein the at least one second sensor comprises an acceleration sensor or a gyro sensor, and

determining whether the determined shaking pattern corresponds to the predetermined condition comprises

determining whether the shaking pattern of the electronic device (101) acquired through the acceleration sensor or the gyro sensor corresponds to a shaking pattern of a situation in which the user gets in a vehicle or a shaking pattern of a situation in which the user is walking.

10. The method according to any one of the previous claims 6-9, wherein the at least one third sensor comprises a first inertial sensor, and

the method comprises:

acquiring a movement distance change amount for at least one axis indicating a preset direction within a space where the electronic device (101) is located, by using the first inertial sensor;

receiving a movement distance change amount of the external electronic device (102) for the at least one axis indicating the preset direction, which is acquired through a second inertial sensor of the external electronic device (102), from the external electronic device (102) by using the wireless communication; and

acquiring the first feature value, based on a difference between the movement distance change amount of the electronic device (101) and the movement distance change amount of the external electronic device (102).

11. The method of claim 10, further comprising:

receiving time information related to the movement distance change amount of the external electronic device (102) from the external electronic device (102) by using the wireless communication; and

correcting the received movement distance change amount of the external electronic device, based on the received time information of the external electronic device (102) and the movement distance change amount of the electronic device (101).

12. The method according to any one of the previous claims 6-11, further comprising:

acquiring data on at least one axis within a space where the external electronic device (102) is located, from the external electronic device (102) by using the wireless communication; and

correcting an axis corresponding to the at least one axis in a space where the electronic device (101) is located, based on the acquired data.

13. The method according to any one of the previous claims 6-12, wherein acquiring the first feature value and the second feature value comprises:

acquiring the first feature value; and

in response to the first feature value exceeding a predetermined threshold value, acquiring the second feature value,

the second feature value comprises a distance between the electronic device (101) and the external electronic device (102) acquired using the at least one third sensor, and

the at least one third sensor comprises an UWB antenna.

**14.** The method according to any one of the previous claims 6-13, wherein controlling the display (710), based on the fatigue level, comprises:

changing a position of a first user interface, UI, displayed on the display, based on the first feature value, a current display position of the first UI, and the second feature value; and

when a distance between the current display position of the first UI and a position to display the first UI is equal to or greater than a predetermined threshold value, displaying a second UI different from the first UI, and

the second UI comprises guide information for reducing the eye fatigue level of the user.

**15.** The method according to any one of the previous claims 6-14, further comprising:

acquiring a cumulative fatigue level accumulated for a predetermined first time, based on the determined fatigue level;

when the cumulative fatigue level exceeds a predetermined threshold value, controlling at least one of a brightness of the display, a scanning rate, and a color for a predetermined second time; and

displaying a third UI for receiving a user's input at a predetermined time, based on the cumulative fatigue level.

**Patentansprüche**

**1.** Elektronische Vorrichtung (101) zur Verringerung der Augenermüdung eines Benutzers, mit:

einer Anzeige (710);
einer drahtlosen Kommunikationsschaltung;
mindestens einem Prozessor (1320) und
einem Speicher, in dem Anweisungen gespeichert sind, die, wenn sie von dem mindestens einen Prozessor (1320) ausgeführt werden, die elektronische Vorrichtung (101) dazu veranlassen:

mithilfe der drahtlosen Kommunikationsschaltung eine betriebsgemäße Verbindung (201) mit einer vom Benutzer getragenen externen elektronischen Vorrichtung (102) herzustellen;
mithilfe mindestens eines ersten Sensors zu ermitteln (202), ob der Blick des Benutzers auf die Anzeige (710) der elektronischen Vorrichtung (101) gerichtet ist;
als Reaktion darauf, dass der Blick des Benutzers auf die elektronische Vorrichtung (101) gerichtet ist, ein Erschütterungsmuster der elektronischen Vorrichtung (101) mithilfe mindestens eines zweiten Sensors zu erfassen;
wenn das erfasste Erschütterungsmuster einer vorgegebenen Bedingung entspricht, mithilfe mindestens eines dritten Sensors einen ersten und einen zweiten Merkmalswert zu erfassen (204), die sich auf eine Positionsänderung zwischen der elektronischen Vorrichtung (101) und der externen elektronischen Vorrichtung (102) beziehen;
den Augenermüdungsgrad des Benutzers anhand des ersten und des zweiten Merkmalswerts zu ermitteln (205) und
die Anzeige (710) anhand des ermittelten Ermüdungsgrads zu steuern (206), um den Augenermüdungsgrad des Benutzers zu reduzieren.

**2.** Elektronische Vorrichtung (101) nach Anspruch 1, wobei die Anweisungen, wenn sie von dem mindestens einen Prozessor (1320) ausgeführt werden, die elektronische Vorrichtung (101) dazu veranlassen:

mithilfe eines Griffsensors zu ermitteln, ob der Benutzer die elektronische Vorrichtung (101) hält;
mithilfe eines Bewegungssensors zu ermitteln, ob die Anzeige (710) dem Gesicht des Benutzers zugewandt ist;
als Reaktion darauf, dass der Benutzer die elektronische Vorrichtung (101) hält und die elektronische Vorrichtung (101) seinem Gesicht zugewandt ist, mithilfe einer Ultrabreitband-Antenne (UWB, ultrawide antenna) den Abstand zur externen elektronischen Vorrichtung (102) zu bestimmen;
mithilfe einer Kamera den Augenbereich des Benutzers zu erkennen;
wenn der Abstand zur externen elektronischen Vorrichtung (102) innerhalb einer festgelegten Entfernung liegt und der Augenbereich des Benutzers erkannt wird, zu feststellen, dass der Blick des Benutzers auf die elektronische Vorrichtung (101) gerichtet ist.

**3.** Elektronische Vorrichtung (101) nach einem der vorhergehenden Ansprüche 1 und 2, wobei der mindestens eine dritte Sensor einen ersten Trägheitssensor umfasst und
wobei die Anweisungen, wenn sie von dem mindestens einen Prozessor (1320) ausgeführt werden, die elektronische Vorrichtung (101) dazu veranlassen:

mithilfe des ersten Trägheitssensors einen Änderungsbetrag der Bewegungsdistanz für mindestens eine Achse zu erfassen, die eine voreingestellte Richtung innerhalb eines Raums repräsentiert, in dem sich die elektronische Vorrichtung (101) befindet;

von der externen elektronischen Vorrichtung (102) mithilfe der drahtlosen Kommunikationsschaltung einen Änderungsbetrag der Bewegungsdistanz der externen elektronischen Vorrichtung (102) für die mindestens eine die voreingestellte Richtung repräsentierende Achse zu empfangen, der durch einen in der externen elektronischen Vorrichtung (102) umfassten zweiten Trägheitssensor erfasst wurde und anhand einer Differenz zwischen dem Änderungsbetrag der Bewegungsdistanz der elektronischen Vorrichtung (101) und dem Änderungsbetrag der Bewegungsdistanz der externen elektronischen Vorrichtung (102) den ersten Merkmalswert zu erfassen.

4. Elektronische Vorrichtung (101) nach einem der vorhergehenden Ansprüche 1 bis 3, wobei der zweite Merkmalswert ein Abstand zwischen der elektronischen Vorrichtung (101) und der externen elektronischen Vorrichtung (102) ist, der mithilfe einer UWB-Antenne erfasst wird, wobei der mindestens eine dritte Sensor vorzugsweise die UWB-Antenne umfasst.

5. Elektronische Vorrichtung (101) nach einem der vorhergehenden Ansprüche 1 bis 4, wobei die Anweisungen, wenn sie von dem mindestens einen Prozessor (1320) ausgeführt werden, die elektronische Vorrichtung (101) dazu veranlassen, anhand des ermittelten Grads der Augenermüdung des Benutzers mindestens eine der folgenden Einstellungen zu steuern: Helligkeit der Anzeige, Farbe, Abtastrate und Anzeigeposition einer auf der Anzeige angezeigten Benutzeroberfläche.

6. Steuerungsverfahren für eine elektronische Vorrichtung (101) zur Reduzierung der Augenermüdung des Benutzers, umfassend:

Verbindung mit einer vom Benutzer getragenen externen elektronischen Vorrichtung (102) mittels drahtloser Kommunikation;

Feststellen, ob der Blick des Benutzers auf die elektronische Vorrichtung (101) gerichtet ist, mithilfe mindestens eines ersten Sensors;

als Reaktion darauf, dass der Blick des Benutzers auf die elektronische Vorrichtung (101) gerichtet ist, Erfassen eines Erschütterungsmusters der elektronischen Vorrichtung mithilfe mindestens eines zweiten Sensors;

wenn das erfasste Erschütterungsmuster einer vorgegebenen Bedingung entspricht Erfassen eines ersten und eines zweiten Merkmalswerts, die sich auf eine Positionsänderung zwischen der elektronischen Vorrichtung (101) und der externen elektronischen Vorrichtung (102) beziehen, mithilfe mindestens eines dritten Sensors;

Bestimmen des Augenermüdungsgrads des Benutzers anhand des ersten und des zweiten Merkmalswerts und

Steuern einer Anzeige (710) der elektronischen Vorrichtung (101) anhand des ermittelten Ermüdungsgrads, um den Augenermüdungsgrad des Benutzers zu reduzieren.

7. Verfahren nach Anspruch 6, wobei der mindestens eine erste Sensor einen Griffsensor und einen Bewegungssensor umfasst, und

wobei der Schritt zum Feststellen, ob der Blick des Benutzers auf die elektronische Vorrichtung (101) gerichtet ist, Folgendes umfasst:

mithilfe des Griffsensors Feststellen, ob der Benutzer die elektronische Vorrichtung (101) hält und

mithilfe des Bewegungssensors Feststellen, ob die Anzeige (710) der elektronischen Vorrichtung (101) dem Gesicht des Benutzers zugewandt ist.

8. Verfahren nach Anspruch 7, ferner umfassend: als Reaktion darauf, dass der Benutzer die elektronische Vorrichtung (101) hält und die Anzeige (710) dem Gesicht des Benutzers zugewandt ist:

Bestimmen des Abstands zur externen elektronischen Vorrichtung (102) mithilfe einer UWB-Antenne;

Erfassen des Augenbereichs des Benutzers mithilfe einer Kamera und

wenn der Abstand zur externen elektronischen Vorrichtung (102) innerhalb einer festgelegten Entfernung liegt und der Augenbereich des Benutzers erfasst wird, Feststellen, dass der Blick des Benutzers auf die elektronische Vorrichtung (101) gerichtet ist.

9. Verfahren nach einem der vorhergehenden Ansprüche 6 bis 8, wobei der mindestens eine zweite Sensor einen Beschleunigungssensor oder einen Gyrosensor umfasst, und

wobei das Ermitteln, ob das ermittelte Erschütterungsmuster der vorgegebenen Bedingung entspricht, Folgendes umfasst:

Ermitteln, ob das durch den Beschleunigungssensor oder den Gyrosensor erfasste Erschütterungsmuster der elektronischen Vorrichtung (101) einem Erschütterungsmuster einer Situation entspricht, in der der Benutzer in ein Fahrzeug steigt, oder einem Erschütterungsmuster einer Situation, in der der Benutzer zu Fuß geht.

**10.** Verfahren nach einem der vorhergehenden Ansprüche 6 bis 9, wobei der mindestens eine dritte Sensor einen ersten Trägheitssensor umfasst, und das Verfahren Folgendes umfasst:

Erfassen eines Änderungsbetrags der Bewegungsdistanz für mindestens eine Achse, die eine voreingestellte Richtung innerhalb eines Raums angibt, in dem sich die elektronische Vorrichtung (101) befindet, mithilfe des ersten Trägheitssensors;
von der externen elektronischen Vorrichtung (102) mittels drahtloser Kommunikation Empfangen eines Änderungsbetrags der Bewegungsdistanz der externen elektronischen Vorrichtung (102) für die mindestens eine die voreingestellte Richtung angebende Achse, der über einen zweiten Trägheitssensor der externen elektronischen Vorrichtung (102) erfasst wurde und
Erfassen des ersten Merkmalswerts anhand einer Differenz zwischen dem Änderungsbetrag der Bewegungsdistanz der elektronischen Vorrichtung (101) und dem Änderungsbetrag der Bewegungsdistanz der externen elektronischen Vorrichtung (102).

**11.** Verfahren nach Anspruch 10, ferner umfassend:

von der externen elektronischen Vorrichtung (102) mittels drahtloser Kommunikation Empfangen von Zeitinformationen, die sich auf den Änderungsbetrag der Bewegungsdistanz der externen elektronischen Vorrichtung (102) beziehen und
Korrigieren des empfangenen Änderungsbetrags der Bewegungsdistanz der externen elektronischen Vorrichtung anhand der empfangenen Zeitinformationen der externen elektronischen Vorrichtung (102) und des Änderungsbetrags der Bewegungsdistanz der elektronischen Vorrichtung (101).

**12.** Verfahren nach einem der vorhergehenden Ansprüche 6 bis 11, ferner umfassend:

Erfassen von von der externen elektronischen Vorrichtung (102) mittels drahtloser Kommunikation gesendeten Daten über mindestens eine Achse in einem Raum, in dem sich die elektronische Vorrichtung (102) befindet und
anhand der erfassten Daten Korrigieren einer der mindestens einen Achse entsprechenden Achse in einem Raum, in dem sich die elektronische Vorrichtung (101) befindet.

**13.** Verfahren nach einem der vorhergehenden Ansprüche 6 bis 12, wobei das Erfassen des ersten und des zweiten Merkmalswerts Folgendes umfasst:

Erfassen des ersten Merkmalswerts und als Reaktion darauf, dass der erste Merkmalswert einen vorgegebenen Schwellenwert überschreitet, Erfassen des zweiten Merkmalswerts, wobei der zweite Merkmalswert einen Abstand zwischen der elektronischen Vorrichtung (101) und der externen elektronischen Vorrichtung (102) umfasst, der mithilfe des mindestens einen dritten Sensors erfasst wird, und der mindestens eine dritte Sensor eine UWB-Antenne umfasst.

**14.** Verfahren nach einem der vorhergehenden Ansprüche 6 bis 13, wobei die Steuerung der Anzeige (710) anhand des Ermüdungsgrads Folgendes umfasst:

Ändern der Position einer ersten Benutzeroberfläche (UI, User Interface), die auf der Anzeige angezeigt wird, anhand des ersten Merkmalswerts, der aktuellen Anzeigeposition der ersten UI und des zweiten Merkmalswerts und
wenn der Abstand zwischen der aktuellen Anzeigeposition der ersten UI und einer Position zum Anzeigen der ersten UI gleich oder größer als ein vorgegebener Schwellwert ist, Anzeigen einer zweiten UI, die sich von der ersten UI unterscheidet, und
wobei die zweite UI Anleitungen zur Reduzierung der Augenermüdung des Benutzers enthält.

**15.** Verfahren nach einem der vorhergehenden Ansprüche 6 bis 14, ferner umfassend:

Erfassen eines kumulierten Ermüdungsgrads, der während einer vorgegebenen ersten Zeit akkumuliert wurde, basierend auf dem ermittelten Ermüdungsgrads;
wenn der kumulierte Ermüdungsgrad einen vorgegebenen Schwellenwert überschreitet, Steuern mindestens eines der folgenden Einstellungen: Helligkeit der Anzeige, Abtastrate und Farbe während einer vorgegebenen zweiten Zeit und
Anzeigen einer dritten UI zum Empfangen einer Benutzereingabe zu einem vorgegebenen Zeitpunkt, basierend auf dem kumulierten Ermüdungsgrad.

## Revendications

**1.** Appareil électronique (101) permettant de réduire la fatigue oculaire d'un utilisateur, comprenant :

un écran (710),

un circuit de communication sans fil,
au moins un processeur (1320), et
une mémoire dans laquelle sont stockées des instructions qui, lorsqu'elles sont exécutées par l'au moins un processeur (1320), amènent l'appareil électronique (101) à :

se connecter de manière fonctionnelle (201), au moyen du circuit de communication sans fil, à un appareil électronique externe (102) porté par l'utilisateur,
déterminer (202), au moyen d'au moins un premier capteur, si l'utilisateur regarde l'écran (710) de l'appareil électronique (101),
en réaction au fait que l'utilisateur regarde l'appareil électronique (101), acquérir, au moyen d'au moins un deuxième capteur, un modèle de secousse de l'appareil électronique (101),
lorsque le modèle de secousse acquis correspond à une condition prédéterminée, acquérir (204), au moyen d'au moins un troisième capteur, une première valeur caractéristique et une deuxième valeur caractéristique qui sont liées à un changement de position entre l'appareil électronique (101) et l'appareil électronique externe (102),
déterminer (205) un niveau de fatigue oculaire de l'utilisateur, compte tenu de la première valeur caractéristique et de la deuxième valeur caractéristique, et
régler (206) l'écran (710), compte tenu du niveau de fatigue déterminé, pour réduire le niveau de fatigue oculaire de l'utilisateur.

2. Appareil électronique (101) selon la revendication 1, dans lequel les instructions, lorsqu'elles sont exécutées par l'au moins un processeur (1320), amènent l'appareil électronique (101) à :

déterminer, au moyen d'un capteur de préhension, si l'utilisateur tient l'appareil électronique (101),
déterminer, au moyen d'un capteur de mouvement, si l'écran (710) est tourné vers le visage de l'utilisateur,
en réaction au fait que l'utilisateur tient l'appareil électronique (101) et que l'appareil électronique (101) est tourné vers le visage de l'utilisateur, déterminer, au moyen une antenne à bande ultralarge (UWB, ultrawide band), une distance par rapport à l'appareil électronique externe (102),
détecter la zone des yeux d'un utilisateur au moyen d'une caméra,
lorsque la distance par rapport à l'appareil électronique externe (102) est dans une plage spécifique et que la zone des yeux de l'utilisateur est détectée, déterminer que l'utilisateur regarde l'appareil électronique (101).

3. Appareil électronique (101) selon l'une quelconque des revendications précédentes 1 et 2, dans lequel l'au moins un troisième capteur comprend un premier capteur inertiel, et
dans lequel les instructions, lorsqu'elles sont exécutées par l'au moins un processeur (1320), amènent l'appareil électronique (101) à :

acquérir, au moyen du premier capteur inertiel, une valeur de changement de distance de mouvement relativement à au moins un axe représentatif d'une direction prédéfinie dans un espace dans lequel se trouve l'appareil électronique (101),
recevoir, en provenance de l'appareil électronique externe (102), par le biais du circuit de communication sans fil, une valeur de changement de distance de mouvement de l'appareil électronique externe (102) relativement à au moins un axe représentatif de la direction prédéfinie, qui est acquise par l'intermédiaire d'un deuxième capteur inertiel compris dans l'appareil électronique externe (102), et
acquérir la première valeur caractéristique, compte tenu d'une différence entre la valeur de changement de distance de mouvement de l'appareil électronique (101) et la valeur de changement de distance de mouvement de l'appareil électronique externe (102).

4. Appareil électronique (101) selon l'une quelconque des revendications précédentes 1 à 3, dans lequel la deuxième valeur caractéristique est une distance entre l'appareil électronique (101) et l'appareil électronique externe (102), qui est acquise au moyen d'une antenne UWB, ledit au moins un troisième capteur comprenant de préférence l'antenne UWB.

5. Appareil électronique (101) selon l'une quelconque des revendications précédentes 1 à 4, dans lequel les instructions, lorsqu'elles sont exécutées par l'au moins un processeur (1320), amènent l'appareil électronique (101) à régler la luminosité de l'écran, la couleur, la vitesse de balayage et/ou la position d'affichage d'une interface utilisateur affichée sur l'écran, compte tenu du niveau de fatigue oculaire déterminé de l'utilisateur.

6. Procédé de commande d'un appareil électronique (101) pour réduire le niveau de fatigue oculaire d'un utilisateur, le procédé comprenant :

une connexion à un appareil électronique externe (102) porté par l'utilisateur, au moyen d'une communication sans fil,

la détermination du fait que l'utilisateur regarde ou non l'appareil électronique (101), au moyen d'au moins un premier capteur,

en réaction au fait que l'utilisateur regarde l'appareil électronique (101), l'acquisition d'un modèle de secousse de l'appareil électronique au moyen d'au moins un deuxième capteur,

lorsque le modèle de secousse acquis correspond à une condition prédéterminée, l'acquisition d'une première valeur caractéristique et d'une deuxième valeur caractéristique qui sont liées à un changement de position entre l'appareil électronique (101) et l'appareil électronique externe (102), au moyen d'au moins un troisième capteur,

la détermination du niveau de fatigue oculaire de l'utilisateur compte tenu de la première valeur caractéristique et de la deuxième valeur caractéristique, et

le réglage d'un écran (710) de l'appareil électronique (101) compte tenu du niveau de fatigue déterminé, pour réduire le niveau de fatigue oculaire de l'utilisateur.

7. Procédé selon la revendication 6, dans lequel l'au moins un premier capteur comprend un capteur de préhension et un capteur de mouvement, et dans laquelle l'étape de détermination du fait que l'utilisateur regarde l'appareil électronique (101) comprend :

la détermination, au moyen du capteur de préhension, du fait que l'utilisateur tient ou non l'appareil électronique (101), et

la détermination, au moyen du capteur de mouvement, du fait que l'écran (710) de l'appareil électronique (101) est tourné ou non vers le visage d'un utilisateur.

8. Procédé selon la revendication 7, comprenant en outre :

en réaction au fait que l'utilisateur tient l'appareil électronique (101) et que l'écran (710) est tourné vers le visage de l'utilisateur :

la détermination de la distance par rapport à l'appareil électronique externe (102) au moyen une antenne UWB,

la détection de la zone des yeux de l'utilisateur au moyen d'une caméra, et

lorsque la distance par rapport à l'appareil électronique externe (102) est dans une plage spécifique et que la zone des yeux de l'utilisateur est détectée, la détermination que l'utilisateur regarde l'appareil électronique (101).

9. Procédé selon l'une quelconque des revendications précédentes 6 à 8, dans lequel l'au moins un deuxième capteur comprend un capteur d'accélération ou un capteur gyroscopique, et

la détermination du fait que le modèle de secousse déterminé correspond ou non à la condition prédéterminée comprend :

la détermination du fait que le modèle de secousse de l'appareil électronique (101) acquis par le biais du capteur d'accélération ou du capteur gyroscopique correspond ou non à un modèle de secousse d'une situation dans laquelle l'utilisateur monte dans un véhicule ou à un modèle de secousse d'une situation dans laquelle l'utilisateur marche.

10. Procédé selon l'une quelconque des revendications précédentes 6 à 9, dans lequel l'au moins un troisième capteur comprend un premier capteur inertiel ; le procédé comprenant :

l'acquisition, au moyen du premier capteur inertiel, d'une valeur de changement de distance de mouvement relativement à au moins un axe indiquant une direction prédéfinie dans un espace dans lequel se trouve l'appareil électronique (101),

la réception, en provenance de l'appareil électronique externe (102) au moyen de la communication sans fil, d'une valeur de changement de distance de mouvement de l'appareil électronique externe (102) relativement à au moins un axe indiquant la direction prédéfinie, qui est acquise par un deuxième capteur inertiel de l'appareil électronique externe (102), et

l'acquisition de la première valeur caractéristique compte tenu d'une différence entre la valeur de changement de distance de mouvement de l'appareil électronique (101) et la valeur de changement de distance de mouvement de l'appareil électronique externe (102).

11. Procédé selon la revendication 10, comprenant en outre :

la réception d'informations temporelles relatives à la valeur de changement de distance de déplacement concernant l'appareil électronique externe (102) à partir de l'appareil électronique externe (102), au moyen la communication sans fil, et

la correction de la valeur de changement de distance de mouvement reçue concernant l'appareil électronique externe, compte tenu des informations temporelles reçues concernant l'appareil électronique externe (102) et de la valeur de changement de distance de mouvement concernant l'appareil électronique (101).

12. Procédé selon l'une quelconque des revendications précédentes 6 à 11, comprenant en outre :

l'acquisition de données sur au moins un axe dans un espace dans lequel se trouve l'appareil électronique externe (102), en provenance de l'appareil électronique externe (102) au moyen la communication sans fil, et

la correction d'un axe correspondant à l'au moins un axe dans un espace dans lequel se trouve l'appareil électronique (101), compte tenu des données acquises.

**13.** Procédé selon l'une quelconque des revendications précédentes 6 à 12, dans lequel l'acquisition de la première valeur caractéristique et de la deuxième valeur caractéristique comprend :

l'acquisition de la première valeur caractéristique, et

en réaction au fait que la première valeur caractéristique dépasse une valeur seuil prédéterminée, l'acquisition de la deuxième valeur caractéristique ;

ladite deuxième valeur caractéristique comprenant une distance entre l'appareil électronique (101) et l'appareil électronique externe (102) acquise au moyen de l'au moins un troisième capteur, et ledit au moins un troisième capteur comprenant une antenne UWB.

**14.** Procédé selon l'une quelconque des revendications précédentes 6 à 13, dans lequel le réglage de l'écran (710) compte tenu du niveau de fatigue comprend :

la modification de la position d'une première interface utilisateur affichée sur l'écran, compte tenu de la première valeur caractéristique, de la position d'affichage actuelle de la première UI et de la deuxième valeur caractéristique, et

lorsque la distance entre la position d'affichage actuelle de la première interface utilisateur et la position destinée à l'affichage de la première interface utilisateur est égale ou supérieure à une valeur seuil prédéterminée, l'affichage d'une deuxième interface utilisateur différente de la première interface utilisateur ;

ladite deuxième interface utilisateur comprenant des informations instructives visant à réduire le niveau de fatigue oculaire de l'utilisateur.

**15.** Procédé selon l'une quelconque des revendications précédentes 6 à 14, comprenant en outre :

l'acquisition d'un niveau de fatigue cumulé pendant une première durée prédéterminée, compte tenu du niveau de fatigue déterminé,

lorsque le niveau de fatigue cumulé dépasse une valeur seuil prédéterminée, le réglage de la luminosité de l'écran, la vitesse de balayage et la couleur pendant une deuxième durée prédéterminée, et

l'affichage d'une troisième interface utilisateur permettant de recevoir une entrée utilisateur, à une heure prédéterminée, compte tenu du niveau de fatigue cumulé.

FIG.1

START

201
CONNECT WITH EXTERNAL ELECTRONIC
DEVICE BY USING WIRELESS COMMUNICATION

202
DETERMINE
WHETHER USER IS
GAZING AT ELECTRONIC DEVICE BY
USING AT LEAST ONE FIRST
SENSOR

NO →

YES

203
DETERMINE
WHETHER ACQUIRED
SHAKING PATTERN OF ELECTRONIC
DEVICE CORRESPONDS TO
PREDETERMINED CONDITION
BY USING AT LEAST ONE
SECOND SENSOR

NO →

YES

204
ACQUIRE FIRST FEATURE VALUE AND SECOND
FEATURE VALUE BY USING AT LEAST ONE THIRD
SENSOR

205
ACQUIRE EYE FATIGUE LEVEL OF USER,
BASED ON FIRST FEATURE VALUE AND
SECOND FEATURE VALUE

206
CONTROL DISPLAY, BASED ON ACQUIRED
FATIGUE LEVEL

END

FIG.2

311

ALIGN AXIS BETWEEN ELECTRONIC
DEVICE AND EXTERNAL ELECTRONIC DEVICE,
AND SYNCHRONIZE TIME INFORMATION

312

ACQUIRE FIRST FEATURE VALUE, BASED ON
MOVEMENT DISTANCE CHANGE AMOUNT FOR
AT LEAST ONE AXIS OF ELECTRONIC DEVIE
AND MOVEMENT DISANCE CHANGE AMOUNT
FOR AT LEAST ONE AXIS OF EXTERNAL
ELECTRONIC DEVICE

313

DETERMINE
WHETHER MOVEMENT
DISTANCE CHANGE AMOUNT FOR
Z AXIS OF ELECTRONIC DEVICE IS
EQUAL TO OR GREATER THAN
FIRST THRESHOLD
VALUE

NO

YES

314

ACQUIRE SECOND FEATURE VALUE BY
USING UWB ANTENNA

205

FIG.3

FIG.4A

FIG.4B

FIG.4C

FIG.4D

204

DETERMINE
WHETHER FIRST FEATURE
VALUE IS EQUAL TO OR GREATER
THAN SECOND THRESHOLD
VALUE

511

NO

YES

513

CONTROL DISPLAY POSITION OF
FIRST UI DISPLAYED ON DISPLAY

FIG.5

FIG.6

FIG.7

205

ACQUIRE CUMULATIVE FATIGUE LEVEL,
BASED ON FATIGUE LEVEL ACQUIRED FOR
PREDETERMINED FIRST TIME
821

DEDTERMINE
WHETHER CUMULATIVE
FATIGUE LEVEL IS EQUAL TO OR
GREATER THAN THIRD
THRESHOLD VALUE
823

NO

YES

CONTROL AT LEAST SOME OF BRIGHTNESS
OF DISPLAY, SCANNING RATE AND COLOR
FOR PREDETERMINED SECOND TIME
825

FIG.8

204

901
INPUT FIRST FEATURE VALUE AND
SECOND FEATURE VALUE TO OUTPUT
CONTROL MODEL

903
CONTROL DISPLAY POSITION OF FIRST
UI DISPLAYED ON DISPLAY, BASED ON
OUTPUT VALUE OF OUTPUT CONTROL
MODEL

905
DETERMINE
WHETHER SHAKING PATTERN
OF ELECRONIC DEVICE ACQUIRED
USING AT LEAST ONE SECOND
SENSOR CORRESPONDS TO
PREDETERMINED
CONDITION

YES

NO

907
DEACTIVATE OUTPUT CONTROL MODEL
AND UWB ANTENNA

FIG.9

710   1030   701

IT SEEMS THAT SHAKING
OF VEHICLE IS CURRENTLY
TOO GREAT DURING VEHICLE
MOVEMENT / WALKING

WHY DON'T YOU PUT
SMARTPHONE DOWN FOR
A WHILE FOR 000'S EYE
HEALTH?

FIG.10

710          1131                     710          1132
                          701                                701

AS RESULT OF MONITORING
000'S EYE HEALTH DURING
THE DAY,
IT SEEMS THAT EYE FATIGUE
IS SLIGHTLY HIGH DUE TO
USE OF MOBILE TERMINAL
FOR LONG TIME WHILE
RIDING IN VEHICLE

METHOD FOR REDUCING
EYE FATIGUE

VIEW /  LATER

① WARM COMPRESS METHOD

② EYE EXERCISE METHOD

. . .

# FIG.11

710 1230 701

AS RESULT OF MONITORING
000'S EYE HEALTH DURING
THE DAY,

IT IS HIGHLY LIKELY THAT
EYE FATIGUE WILL INCREASE
WHEN USING MOBILE
TERMINAL BEFORE SLEEPING

PLEASE REFRAIN FROM
USING MOBILE TERMINAL,
AND USE EYE PROTECTION
MODE WHEN USING IT

EYE PROTECTION MODE
EXECUTED?  Y/N

FIG.12

1300

ELECTRONIC DEVICE (1301)

| INPUT MODULE (1350) |
| SOUND OUTPUT MODULE (1355) |

DISPLAY MODULE (1360)

MEMORY (1330)

VOLATILE MEMORY (1332)

NON-VOLATILE MEMORY (1334)

INTERNAL MEMORY (1336)

EXTERNAL MEMORY (1338)

PROGRAM (1340)

APPLICATION (1346)

MIDDLEWARE (1344)

OPERATING SYSTEM (1342)

BATTERY (1389)

POWER MANAGEMENT MODULE (1388)

PROCESSOR (1320)

MAIN PROCESSOR (1321)

AUXILIARY PROCESSOR (1323)

COMMUNICATION MODULE (1390)

WIRELESS COMMUNICATION MODULE (1392)

WIRED COMMUNICATION MODULE (1394)

SUBSCRIBER IDENTIFICATION MODULE (1396)

ANTENNA MODULE (1397)

AUDIO MODULE (1370)

HAPTIC MODULE (1379)

SENSOR MODULE (1376)

CAMERA MODULE (1380)

INTERFACE (1377)

CONNECTING TERMINAL (1378)

SECOND NETWORK (1399)

ELECTRONIC DEVICE (1304)

FIRST NETWORK (1398)

ELECTRONIC DEVICE (1302)

SERVER (1308)

# FIG.13

**EP 4 270 160 B1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 10775883 B2

- KR 101540161 B1